# EUROPÄISCHE PATENTSCHRIFT

(11) **EP 2 635 577 B1**
(45) Veröffentlichungstag und Bekanntmachung des Hinweises auf die Patenterteilung: **01.10.2014**
(21) Anmeldenummer: 11788085.6
(22) Anmeldetag: 03.11.2011
(51) Int. Cl.: C07D 471/04, A61K 31/437, A61P 9/00

(54) **SUBSTITUIERTE 6-FLUOR-1H-PYRAZOLO[4,3-B]PYRIDINE UND IHRE VERWENDUNG**
SUBSTITUTED 6-FLUORO-1H-PYRAZOLO[4,3-B]PYRIDINES AND USE THEREOF
6-FLUORO-1H-PYRAZOLO[4,3-B]PYRIDINES SUBSTITUÉES ET LEUR UTILISATION

(30) Priorität: 04.11.2010 DE 102010043379
(43) Veröffentlichungstag der Anmeldung: 11.09.2013
(73) Patentinhaber: Bayer Intellectual Property GmbH, 40789 Monheim (DE)
(72) Erfinder: FOLLMANN, Markus, 50859 Köln (DE); STASCH, Johannes-Peter, 42651 Solingen (DE); REDLICH, Gorden, 44799 Bochum (DE); ACKERSTAFF, Jens, 10437 Berlin (DE); GRIEBENOW, Nils, 41541 Dormagen (Zons) (DE); KNORR, Andreas, 40699 Erkrath (DE); WUNDER, Frank, 42117 Wuppertal (DE); LI, Volkhart, Min-Jian, 42553 Velbert (DE); MITTENDORF, Joachim, 42113 Wuppertal (DE); SCHLEMMER, Karl-Heinz, 42113 Wuppertal (DE); JAUTELAT, Rolf, 42781 Haan (DE)
(74) Vertreter: BIP Patents
(86) Internationale Anmeldenummer: PCT/EP2011/069346
(87) Internationale Veröffentlichungsnummer: WO 2012/059549

(56) Entgegenhaltungen:
- WO-A1-2008/031513
- WO-A1-2010/079120

## Beschreibung

Die vorliegende Anmeldung betrifft neue substutuierte 6-Fluor-1H-Pyrazolo[4,3-b]pyridine, Verfahren zu ihrer Herstellung, ihre Verwendung allein oder in Kombinationen zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von Herz-Kreislauf-Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, an das Eisen-Zentralatom des Häms zu binden, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion, der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Arteriosklerose, Angina pectoris, Herzinsuffizienz, Myokardinfarkt, Thrombosen, Schlaganfall und sexueller Dysfunktion führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriff am Eisen-Zentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxymethyl-2'-furyl)-1-benzylindazol [YC-1; Wu et al., Blood 84 (1994), 4226; Mülsch et al., Brit. J. Pharmacol. 120 (1997), 681], Fettsäuren [Goldberg et al., J. Biol. Chem. 252 (1977), 1279], Diphenyliodonium-hexafluorophosphat [Pettibone et al., Eur. J. Pharmacol. 116 (1985), 307], Isoliquiritigenin [Yu et al., Brit. J. Pharmacol. 114 (1995), 1587] sowie verschiedene substituierte Pyrazol-Derivate (WO 98/16223).

WO 2008/031513 und WO2010/079120 offenbaren unter anderem 1H-Pyrazolo[4,3-b]pyridine als Stimulatoren der löslichen Guanylatcyclase zur Behandlung von Herzkreislauferkrankungen. In WO 2005/030121 werden annellierte Pyrazole zur Behandlung von Krebserkrankungen beschrieben. WO 2011/119518 und WO 2011/115804 offenbaren Carbamat-substituierte Pyrimidine zur Behandlung von Herzkreislauferkrankungen.

Aufgabe der vorliegenden Erfindung war die Bereitstellung neuer Substanzen, die als Stimulatoren der löslichen Guanylatcyclase wirken und ein gleiches oder verbessertes therapeutisches Profil gegenüber den aus dem Stand der Technik bekannten Verbindungen aufweisen, wie beispielsweise hinsichtlich ihrer *in-vivo* Eigenschaften, wie beispielsweise ihrem pharmakokinetischem und/ oder pharmakodynamischem Verhalten und/oder ihrer Dosis-Wirkungsbeziehung.

Gegenstand der vorliegenden Erfindung sind Verbindungen der allgemeinen Formel (I) 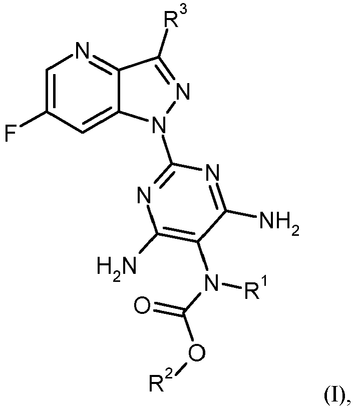 in welcher
- R¹: für Wasserstoff oder (C₁-C₄)-Alkyl steht,
wobei (C₁-C₄)-Alkyl mit ein oder zwei Substituenten unabhängig voneinander ausgewählt aus der Gruppe Fluor und Trifluormethyl substituiert sein kann,
- R²: für (C₁-C₄)-Alkyl, (C₃-C₇)-Cycloalkyl oder einen 4- bis 7-gliedrigen Heterocyclus steht,
wobei (C₁-C₄)-Alkyl mit ein oder zwei Substituenten unabhängig voneinander ausgewählt aus der Gruppe Fluor, Trifluormethyl und (C₃-C₇)-Cycloalkyl substituiert sein kann,
- R³: für (C₁-C₆)-Alkyl oder Benzyl steht,
wobei (C₁-C₆)-Alkyl mit einem Substituenten Trifluormethyl substituiert ist,
wobei (C₁-C₆)-Alkyl mit 1 bis 3 Substituenten Fluor substituiert sein kann,
und
wobei Benzyl mit 1 bis 3 Substituenten Fluor substituiert ist,
sowie ihre *N*-Oxide, Salze, Solvate, Salze der *N*-Oxide und Solvate der *N*-Oxide und Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor Brom und Iod wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Außerdem umfasst die vorliegende Erfindung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
Alkyl steht im Rahmen der Erfindung für einen linearen oder verzweigten Alkylrest mit 1 bis 4 Kohlenstoffatomen. Beispielhaft und vorzugsweise seien genannt: Methyl, Ethyl, n-Propyl, Isopropyl, n-Butyl, iso-Butyl, 1-Methylpropyl, tert.-Butyl.
Cycloalkyl steht im Rahmen der Erfindung für einen monocyclischen, gesättigten Carbocyclus mit 3 bis 7 Ring-Kohlenstoffatomen. Beispielhaft und vorzugsweise seien genannt: Cyclopropyl, Cyclobutyl, Cyclopentyl, Cyclohexyl und Cycloheptyl.
Heterocyclus steht im Rahmen der Erfindung für einen gesättigten Heterocyclus mit insgesamt 4 bis 7 Ringatomen, der ein oder zwei Ring-Heteroatome aus der Reihe N, O und/oder S enthält und über ein Ring-Kohlenstoffatom verknüpft ist. Beispielhaft seien genannt: Azetidinyl, Pyrrolidinyl, Pyrazolidinyl, Tetrahydrofuranyl, Piperidinyl, Piperazinyl, Tetrahydropyranyl, Morpholinyl, Thiomorpholinyl und Azepanyl. Bevorzugt sind Azetidinyl, Pyrrolidinyl, Tetrahydrofuranyl, Piperidinyl, Piperazinyl, Tetrahydropyranyl und Morpholinyl. Besonders sind Azetidinyl, Pyrrolidinyl, Piperidinyl und Morpholinyl.
Halogen steht im Rahmen der Erfindung für Fluor, Chlor, Brom und Iod.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt.

Bevorzugt sind im Rahmen der vorliegenden Erfindung Verbindungen der Formel (I), in welcher
- R¹: für Wasserstoff, Methyl oder Ethyl steht,
wobei Methyl mit einem Substituenten Trifluormethyl substituiert sein kann,
- R²: für Methyl, Ethyl, iso-Propyl, Cyclopropyl, Cyclobutyl oder Oxetanyl steht,
wobei Methyl und Ethyl mit ein oder zwei Substituenten unabhängig voneinander ausgewählt aus der Gruppe Fluor, Trifluormethyl und Cyclopropyl substituiert sein können,
- R³: für 3,3,3-Trifluorprop-1-yl, 3,3,4,4,4-Pentafluorbut-1-yl oder Benzyl steht,
wobei Benzyl mit 1 oder 2 Substituenten Fluor substituiert ist,
sowie ihre Salze, Solvate und Solvate der Salze.

Besonders bevorzugt sind im Rahmen der vorliegenden Erfindung Verbindungen der Formel (I), in welcher
- R¹: für Wasserstoff, Methyl, Ethyl oder 2,2,2-Trifluorethyl steht,
- R²: für Methyl, Ethyl oder Cyclopropylmethyl steht,
- R³: für 2-Fluorbenzyl steht,
sowie ihre Salze, Solvate und Solvate der Salze.

Bevorzugt sind im Rahmen der vorliegenden Erfindung auch Verbindungen der Formel (I), in welcher
- R¹: für Wasserstoff, Methyl oder Ethyl steht,
wobei Methyl mit einem Substituenten Trifluormethyl substituiert sein kann,
- R²: für Methyl, Ethyl, iso-Propyl, Cyclopropyl, Cyclobutyl oder Oxetanyl steht,
wobei Methyl und Ethyl mit ein oder zwei Substituenten unabhängig voneinander ausgewählt aus der Gruppe Fluor, Trifluormethyl und Cyclopropyl substituiert sein können,
- R³: für 2-Fluorbenzyl steht,
sowie ihre Salze, Solvate und Solvate der Salze.

Bevorzugt sind im Rahmen der vorliegenden Erfindung auch Verbindungen der Formel (I), in welcher
- R¹: für Wasserstoff, Methyl, Ethyl oder 2,2,2-Trifluorethyl steht,
sowie ihre Salze, Solvate und Solvate der Salze.

Bevorzugt sind im Rahmen der vorliegenden Erfindung auch Verbindungen der Formel (I), in welcher
- R²: für Methyl, Ethyl oder Cyclopropylmethyl steht,
sowie ihre Salze, Solvate und Solvate der Salze.

Bevorzugt sind im Rahmen der vorliegenden Erfindung auch Verbindungen der Formel (I), in welcher
- R³: für 2-Fluorbenzyl steht,
sowie ihre Salze, Solvate und Solvate der Salze.

Bevorzugt sind im Rahmen der vorliegenden Erfindung auch Verbindungen der Formel (I), in welcher
- R³: für 3,3,3-Trifluorprop-1-yl oder 3,3,4,4,4-Pentafluorbut-1-yl steht,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I) dadurch gekennzeichnet, dass man eine Verbindung der Formel (II) 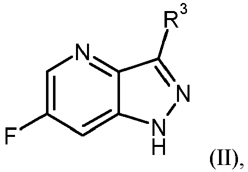 in welcher R³ die oben angegebene Bedeutung hat,
[A] in einem inerten Lösungsmittel in Gegenwart einer geeigneten Base mit der Verbindung der Formel (III) 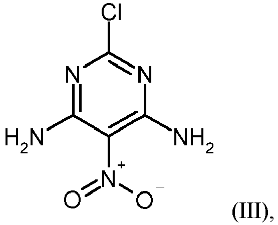 zu einer Verbindung der Formel (IV) 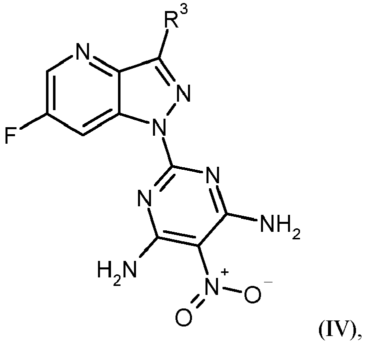 in welcher R³ die oben angegebene Bedeutung hat,
   umsetzt, diese dann in einem inerten Lösungsmittel mit einem geeigneten Reduktionsmittel zu einer Verbindung der Formel (V) 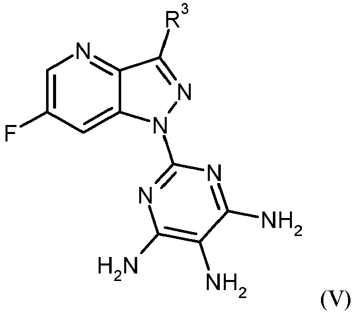 in welcher R³ die oben angegebene Bedeutung hat,
   reduziert, diese im Anschluss in Gegenwart einer geeigneten Base mit oder ohne Lösungsmittel mit einer Verbindung der Formel (VI) 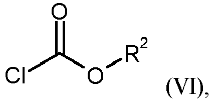 in welcher R² die oben angegebene Bedeutung hat,
   zu einer Verbindung der Formel (I-A) 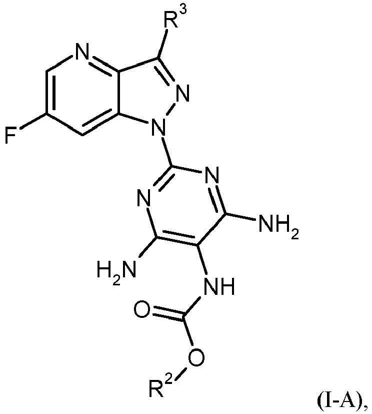 in welcher R² und R³ jeweils die oben angegebenen Bedeutungen haben,
   umsetzt,
   oder
[B] eine Verbindung der Formel (II) in einem inerten Lösungsmittel unter sauren Bedingungen mit Aminoacetonitril in eine Verbindung der Formel (VII) 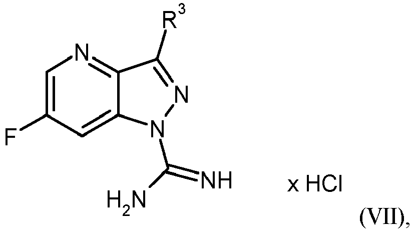 in welcher R³ die oben angegebene Bedeutung hat,
   überführt, diese im Folgenden in einem inerten Lösungsmittel in Gegenwart einer geeigneten Base mit der Verbindung der Formel (VIII) 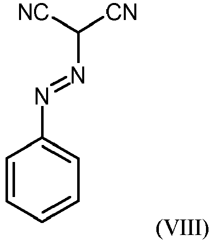 zu einer Verbindung der Formel (IX) 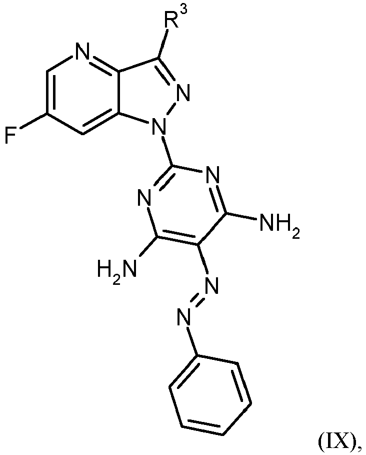 in welcher R³ die oben angegebene Bedeutung hat,
   reagiert, und diese anschliessend in einem inertem Lösungsmittel in Gegenwart eines geeigneten Reduktionsmittels zur Verbindung (V) reduziert, und diese im Folgenden nach Verfahren [A] weiter zu einer Verbindung (I-A) umsetzt,
   oder
[C] eine Verbindung der Formel (I-A) in einem inerten Lösungsmittel mit einer Verbindung der Formel (X)

   R¹-X¹ (X),

   in welcher R¹ die oben angebene Bedeutung hat und
   - X¹: für eine geeignete Abgangsgruppe, wie beispielsweise Halogen, Tosylat oder Mesylat, insbesondere Brom oder Iod, steht,
   zu einer Verbindung der Formel (I-B) 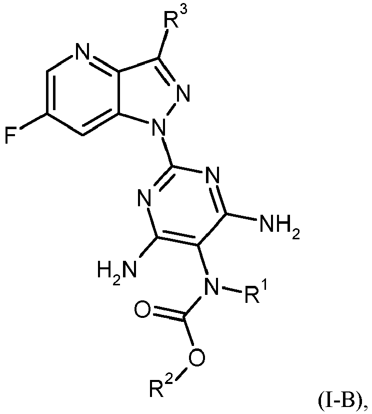 in welcher R¹, R² und R³ jeweils die oben angegebenen Bedeutungen haben,
   umsetzt,
   gegebenenfalls die resultierenden Verbindungen der Formeln (I-A) und (I-B) gegebenenfalls mit den entsprechenden (i) Lösungsmitteln und/oder (ii) Säuren oder Basen in ihre Solvate, Salze und/oder Solvate der Salze überführt.

Die Verbindungen der Formeln (I-A) und (I-B) bilden zusammen die Gruppe der erfindungsgemäßen Verbindungen der Formel (I).

Inerte Lösungsmittel für den Verfahrensschritt (II) + (III) → (IV) sind beispielsweise Alkohole wie Methanol, Ethanol, n-Propanol, Isopropanol, n-Butanol oder tert.-Butanol, Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N*,*N'*-Dimethylpropylenharnstoff (DMPU), Dimethylacetamid, *N*-Methylpyrrolidon (NMP), Pyridin, Acetonitril, Sulfolan oder auch Wasser. Ebenso ist es möglich, Gemische der genannten Lösungsmittel einzusetzen. Bevorzugt ist Dioxan.

Geeignete Basen für den Verfahrensschritt(II) + (III) → (IV) sind Alkalihydride wie Natriumhydrid, Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, Amide wie Natriumamid, Lithium-, Natrium- oder Kalium-bis-(trimethylsilyl)amid oder Lithiumdiisopropylamid, metallorganische Verbindungen wie Butyllithium oder Phenyllithium, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt ist Lithium-bis-(trimethylsilyl)amid oder Natriumhydrid.

Die Reaktion (II) + (III) → (IV) wird im Allgemeinen in einem Temperaturbereich von +20°C bis +180°C, bevorzugt bei +50°C bis +120°C, gegebenenfalls in einer Mikrowelle, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Die Reduktionen (IV) → (V) und (IX) → (V) erfolgen in Gegenwart eines geeigneten Katalysators in einem inerten Lösungsmittel, in einem Temperaturbereich von +20°C bis +40°C unter Wasserstoff-Normaldruck.

Inerte Lösungsmittel für die Reduktionen (IV) → (V) und (IX) → (V) sind beispielsweise Alkohole wie Methanol, Ethanol, n-Propanol, Isopropanol, n-Butanol oder tert.-Butanol, Ether wie Diethylether, Dioxan, Tetrahydrofuran, Glykoldimethylether oder Diethylenglykoldimethylether, oder andere Lösungsmittel wie Dimethylformamid (DMF), Dimethylsulfoxid (DMSO), *N*,*N*'-Dimethylpropylen-harnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt sind DMF und Pyridin.

Geeignete Katalysatoren für die Umsetzungen (IV) → (V) und (IX) → (V) sind beispielsweise Palladium auf Aktivkohle, Platin auf Kohle, Palladiumhydroxid oder Raney-Nickel.

Die Reduktionen (IV) → (V) und (IX) → (V) können alternativ mit einem Metall bzw. Metallsalz wie beispielsweise Eisen, Zink oder Zinn(II)chlorid in einer geeigneten Säure wie beispielsweise Chlorwasserstoff/ Salzsäure, Schwefelsäure, Phosphorsäure oder Essigsäure in einem Temperaturbereich von +20°C bis +140°C erfolgen.

Inerte Lösungsmittel für den Verfahrensschritt (V) + (VI) → (I-A) sind beispielsweise Alkohole wie Methanol, Ethanol, n-Propanol, Isopropanol, n-Butanol oder tert.-Butanol, Ether wie Diethylether, Dioxan, Tetrahydrofuran, Glykoldimethylether oder Diethylenglykoldimethylether, Halogenkohlenwasserstoffe wie Dichlormethan, Trichlormethan, Tetrachlormethan, Trichlorethylen oder Chlorbenzol, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N'-*Dimethylpropylenharnstoff (DMPU), *N*-Methylpyrrolidon (NMP), Acetonitril oder auch Wasser. Ebenso ist es möglich, Gemische der genannten Lösungsmittel einzusetzen. Bevorzugt sind Dimethylformamid und Toluol sowie ein Gemisch aus Dimethylformamid und Toluol.

Geeignete Basen für den Verfahrensschritt (V) + (VI) → (I-A) sind Alkalihydride wie Natriumhydrid, Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, Amide wie Natriumamid, Lithium-, Natrium- oder Kalium-bis-(trimethylsilyl)amid oder Lithiumdiisopropylamid, metallorganische Verbindungen wie Butyllithium oder Phenyllithium, oder organische Amine wie Triethylamin, Diisopropylethylamin, Pyridin, 1,8-Diazabicyclo[5.4.0]undec-7-en (DBU) oder 1,5-Diazabicyclo[4.3.0]-non-5-en (DBN). Bevorzugt ist Pyridin.

Die Reaktion (V) + (VI) → (I-A) wird im Allgemeinen in einem Temperaturbereich von -10°C bis +30°C, bevorzugt bei 0°C bis +20°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für den Verfahrensschritt (VII) + (VIII) → (IX) sind Alkohole wie Methanol, Ethanol, n-Propanol, Isopropanol, n-Butanol oder tert.-Butanol, Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N*,*N*'-Dimethylpropylenharnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt ist DMF.

Inerte Lösungsmittel für den Verfahrensschritt (II) → (VII) sind Alkohole wie Methanol, Ethanol, n-Propanol, Isopropanol, n-Butanol oder tert.-Butanol, Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethyl-formamid (DMF), Dimethylsulfoxid (DMSO), *N*,*N'*-Dimethylpropylenharnstoff (DMPU), *N-*Methylpyrrolidon (NMP), Pyridin oder auch Wasser. Ebenso ist es möglich, Gemische der genannten Lösungsmittel einzusetzen. Bevorzugt ist DMF.

Als Säuren eignen sich beispielsweise anorganische Säuren wie Schwefelsäure, Chlorwasserstoff/ Salzsäure, Polyphosphorsäure oder Phosphorsäure, organische Säuren wie Essigsäure, Trifluoressigsäure, Methansulfonsäure, Toluolsulfonsäure. Bevorzugt ist Chlorwasserstoff/ Salzsäure.

Die Reaktion (II) → (VII) wird im Allgemeinen in einem Temperaturbereich von -10°C bis +30°C, bevorzugt bei 0°C bis +20°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Geeignete Basen für den Verfahrensschritt (VII) + (VIII) → (IX) sind Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt ist Triethylamin.

Die Reaktion (VII) + (VIII) → (IX) wird im Allgemeinen in einem Temperaturbereich von +20°C bis +150°C, bevorzugt bei +80°C bis +120°C, gegebenenfalls in einer Mikrowelle, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Die Verbindung der Formel (VIII) kann analog Literatur L.F.Cavalieri, J.F.Tanker, A.Bendich, J.Am.Chem.Soc., 1949, 71, 533 dargestellt werden.

Inerte Lösungsmittel für den Verfahrensschritt (I-A) + (X) → (I-B) sind beispielsweise Alkohole wie Methanol, Ethanol, n-Propanol, Isopropanol, n-Butanol oder tert.-Butanol, Ether wie Diethylether, Dioxan, Tetrahydrofuran, Glykoldimethylether oder Diethylenglykoldimethylether, Halogenkohlenwasserstoffe wie Dichlormethan, Trichlormethan, Tetrachlormethan, Trichlorethylen oder Chlorbenzol, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N'-*Dimethylpropylenharnstoff (DMPU), *N*-Methylpyrrolidon (NMP), Acetonitril oder auch Wasser. Ebenso ist es möglich, Gemische der genannten Lösungsmittel einzusetzen. Bevorzugt ist THF.

Geeignete Basen für den Verfahrensschritt (I-A) + (X) → (I-B) sind Alkalihydride wie Natriumhydrid, Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, Amide wie Natriumamid, Lithium-, Natrium- oder Kalium-bis-(trimethylsilyl)amid oder Lithiumdiisopropylamid, metallorganische Verbindungen wie Butyllithium oder Phenyllithium, oder organische Amine wie Triethylamin, Diisopropylethylamin, Pyridin, 1,8-Diazabicyclo[5.4.0]undec-7-en (DBU) oder 1,5-Diazabicyclo[4.3.0]-non-5-en (DBN). Bevorzugt ist Lithium-bis-(trimethylsilyl)amid oder Natriumhydrid.

Die Reaktion (I-A) + (X) → (I-B) wird im Allgemeinen in einem Temperaturbereich von -10°C bis +30°C, bevorzugt bei 0°C bis +20°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Die beschriebenen Herstellverfahren werden durch die folgenden Syntheseschemata (Schema 1 bis 3) beispielhaft verdeutlicht:

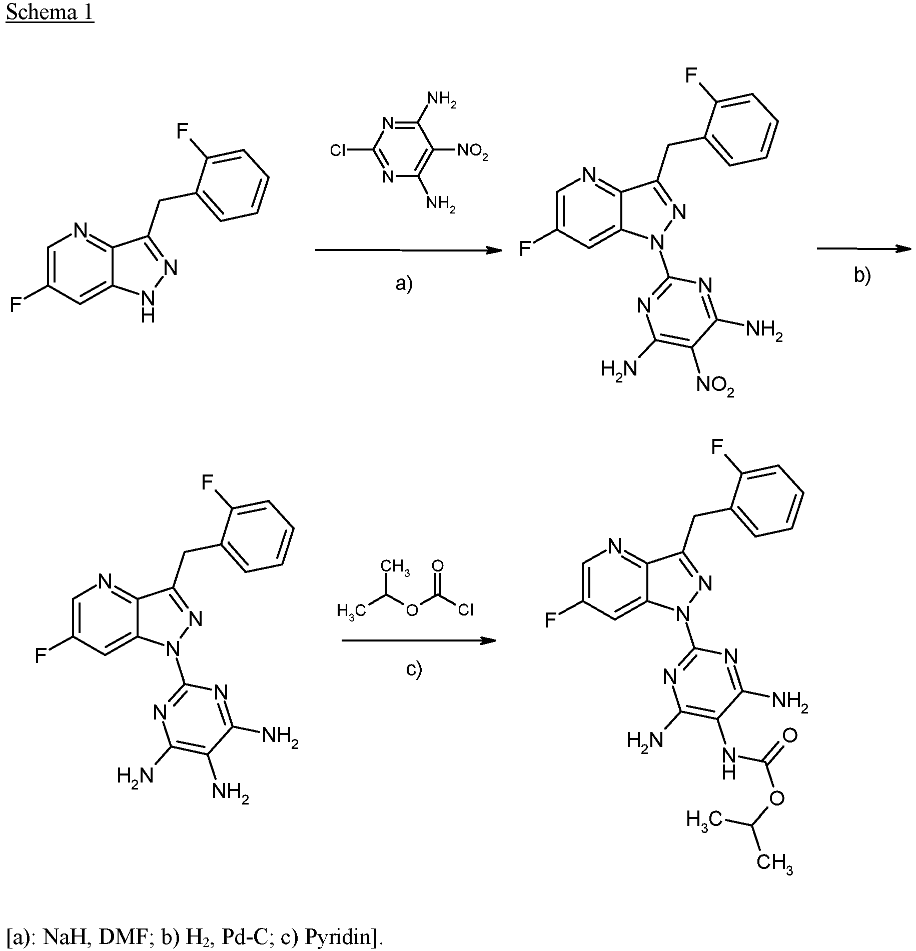

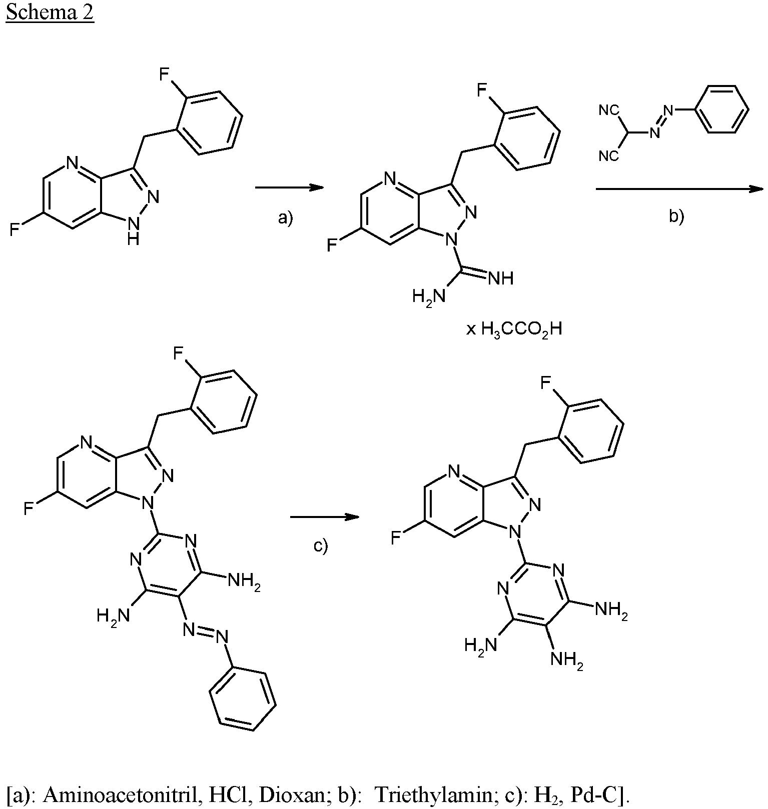

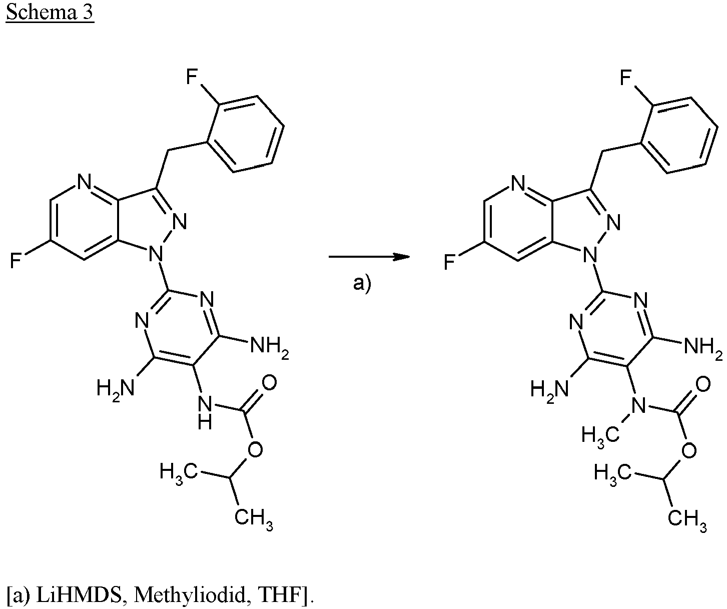

Die Verbindung der Formel (II) kann hergestellt werden, indem man die Verbindung der Formel (XI) 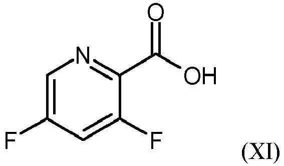 zunächst mit Thionylchlorid in das entsprechende Säurechlorid überführt, dieses dann in einem inerten Lösungsmittel in Gegenwart einer geeigneten Base mit einer Verbindung der Formel (XII) 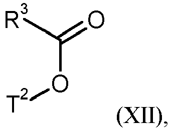 in welcher R³ die oben angegebene Bedeutung hat und
- T²: für (C₁-C₄)-Alkyl steht,
zu einer Verbindung der Formel (XIII) umsetzt 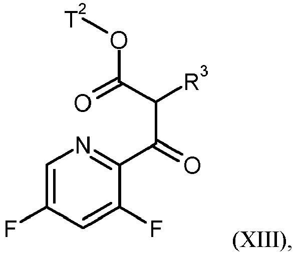 in welcher R³ und T² jeweils die zuvor angegebenen Bedeutungen haben,
diese anschliessend in einem inerten Lösungsmittel zu einer Verbindung der Formel (XIV) 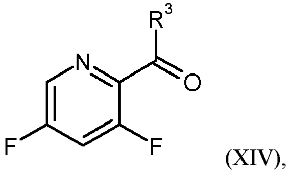 in welcher R³ die oben angegebene Bedeutung hat,
decarboxyliert, und diese dann in einem inerten Lösungsmittel mit Hydrazinhydrat cyclisiert.

Inerte Lösungsmittel für den Verfahrensschritt (XI) + (XII) → (XIII) sind beispielsweise Ether wie Diethylether, Dioxan, Tetrahydrofuran, Glykoldimethylether oder Diethylenglykoldimethylether, Halogenkohlenwasserstoffe wie Dichlormethan, Trichlormethan, Tetrachlormethan, Trichlorethylen oder Chlorbenzol, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N*,*N*'-Dimethylpropylenharnstoff (DMPU), *N*-Methylpyrrolidon (NMP) oder Acetonitril. Ebenso ist es möglich, Gemische der genannten Lösungsmittel einzusetzen. Bevorzugt ist THF.

Geeignete Basen für den Verfahrensschritt (XI) + (XII) → (XIII) sind Alkalihydride wie Natriumhydrid, Alkalialkoholate wie Natrium- oder Kaliummethanolat, Natrium- oder Kaliumethanolat oder Kalium-tert.-butylat, Amide wie Natriumamid, Lithium-, Natrium- oder Kalium-bis-(trimethylsilyl)amid oder Lithiumdiisopropylamid, metallorganische Verbindungen wie Butyllithium oder Phenyllithium. Bevorzugt ist Lithium-bis-(trimethylsilyl)amid.

Die Reaktion (XI) + (XII) → (XIII) wird im Allgemeinen in einem Temperaturbereich von -78°C bis +30°C, bevorzugt bei -78°C bis +20°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Die Decarboxylierung (XIII) → (XIV) erfolgt in einem inerten Lösungsmittel mit Lithium- oder Natriumchlorid.

Inerte Lösungsmittel für die Decarboxylierung (XIII) → (XIV) sind Alkohole wie Methanol, Ethanol, n-Propanol, Isopropanol, n-Butanol, tert.-Butanol oder 1,2-Ethandiol, Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N*,*N*'-Dimethylpropylenharnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt ist ein Gemisch aus DMSO und Wasser.

Die Reaktion (XIII) → (XIV) wird im Allgemeinen in einem Temperaturbereich von +100°C bis +200°C, bevorzugt bei +120°C bis +160°C, gegebenenfalls in einer Mikrowelle, durchgeführt. Die Umsetzung kann bei normalem, erhöhtem oder bei erniedrigtem Druck erfolgen (z.B. von 0.5 bis 5 bar).

Inerte Lösungsmittel für den Verfahrensschritt (XIV) → (II) sind Alkohole wie Methanol, Ethanol, n-Propanol, Isopropanol, n-Butanol, tert.-Butanol oder 1,2-Ethandiol, Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N*,*N'*-Dimethylpropylenharnstoff (DMPU), *N*-Methylpyrrolidon (NMP), Pyridin oder Acetonitril. Ebenso ist es möglich, Gemische der genannten Lösungsmittel einzusetzen. Bevorzugt ist Pyridin gegebenenfalls unter Zusatz katalytischer Mengen von N,N-Dimethylaminopyridin.

Die Reaktion (XIV) → (II) wird im Allgemeinen in einem Temperaturbereich von +60°C bis +200°C, bevorzugt bei +120°C bis +180°C, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Das zuvor beschriebene Verfahren wird durch das nachfolgende Schema (Schema 4) beispielhaft verdeutlicht:

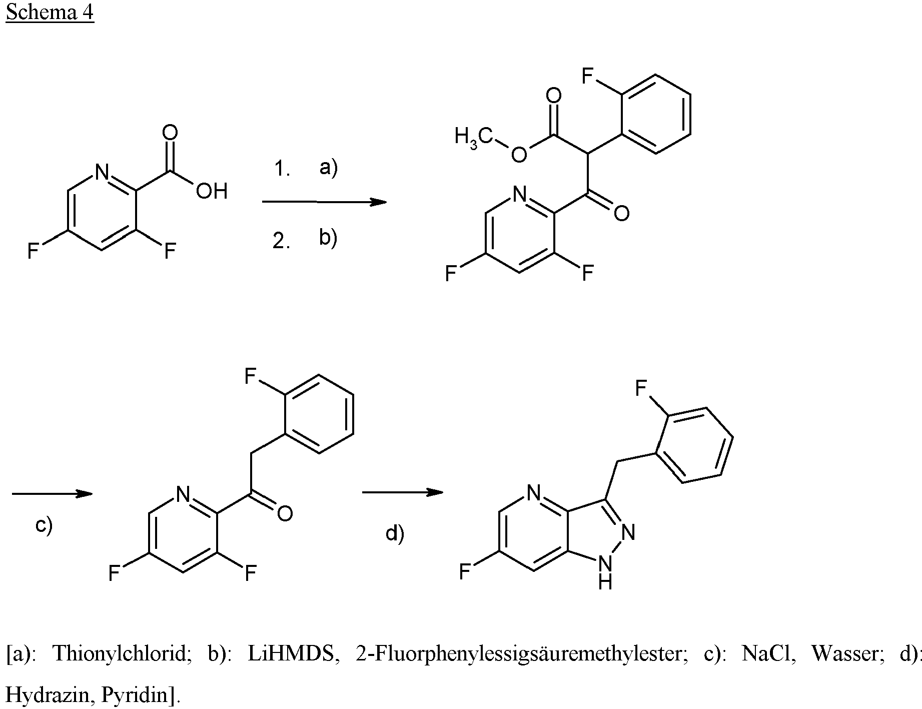

Die erfindungsgemäßen Verbindungen wirken als Stimulatoren der löslichen Guanylatcyclase, besitzen wertvolle pharmakologische Eigenschaften und weisen ein verbessertes pharmakokinetisches und/ oder pharmakodynamisches Profil auf. Sie sich daher zur Behandlung und/ oder Prophylaxe von Erkrankungen bei Menschen und Tieren.

Die erfindungsgemäßen Verbindungen bewirken eine Gefäßrelaxation und eine Hemmung der Thrombozytenaggregation und führen zu einer Blutdrucksenkung sowie zu einer Steigerung des koronaren Blutflusses. Diese Wirkungen sind über eine direkte Stimulation der löslichen Guanylatcyclase und einen intrazellulären cGMP-Anstieg vermittelt. Außerdem verstärken die erfindungsgemäßen Verbindungen die Wirkung von Substanzen, die den cGMP-Spiegel steigern, wie beispielsweise EDRF (endothelium-derived relaxing factor), NO-Donatoren, Protoporphyrin IX, Arachidonsäure oder Phenylhydrazin-Derivate.

Die erfindungsgemäßen Verbindungen können daher in Arzneimitteln zur Behandlung und/oder Prophylaxe von kardiovaskulären Erkrankungen wie beispielsweise Bluthochdruck, akute und chronische Herzinsuffizienz, koronare Herzerkrankung, stabile und instabile Angina pectoris, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden Grad I-III (AB-Block I-III), supraventrikuläre Tachyarrhythmie, Vorhofflimmern, Vorhoffflattern, Kammerflimmern, Kammerflattern, ventrikuläre Tachyarrhytmie, Torsade de pointes-Tachykardie, Extrasystolen des Vorhoffs und des Ventrikels, AV-junktionale Extrasystolen, Sick-Sinus Syndrom, Synkopen, AV-Knoten-Reentrytachykardie, Wolff-Parkinson-White-Syndrom, von akutem Koronarsyndrom (ACS), autoimmune Herzerkrankungen (Perikarditis, Endokarditis, Valvolitis, Aortitis, Kardiomyopathien), kardiogenem Schock, Aneurysmen, Boxerkardiomyopathie (premature ventricular contraction (PVC)), zur Behandlung und/oder Prophylaxe von thromboembolischen Erkrankungen und Ischämien wie myokardiale Ischämie, Myokardinfarkt, Hirnschlag, Schock, Herzhypertrophie, transistorischen und ischämischen Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, peripheren Durchblutungsstörungen, Reperfusionsschäden, arterielle und venöse Thrombosen, Mikroalbuminurie, Herzmuskelschwäche, endotheliale Dysfunktion, zur Verhinderung von Restenosen wie nach Thrombolysetherapien, percutan-transluminalen Angioplastien (PTA), transluminalen Koronarangioplastien (PTCA), Herztransplantationen und Bypass-Operationen, sowie mikro- und makrovaskuläre Schädigungen (Vasculitis), erhöhte Spiegel von Fibrinogen und von LDL geringer Dichte sowie erhöhte Konzentrationen von Plasminogenaktivator-Inhibitor 1 (PAI-1), sowie zur Behandlung und/oder Prophylaxe von erektiler Dysfunktion und weiblicher sexueller Dysfunktion eingesetzt werden.

Im Sinne der vorliegenden Erfindung umfasst der Begriff Herzinsuffizienz auch spezifischere oder verwandte Krankheitsformen wie akut dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz.

Darüber hinaus können die erfindungsgemäßen Verbindungen auch zur Behandlung und/oder Prophylaxe von Arteriosklerose, Lipidstoffwechselstörungen, Hypolipoproteinämien, Dyslipidämien, Hypertriglyceridämien, Hyperlipidämien, Hypercholesterolämien, Abetelipoproteinämie, Sitosterolämie, Xanthomatose, Tangier Krankheit, Fettsucht (Adipositas), Fettleibigkeit (Obesitas) und von kombinierten Hyperlipidämien sowie des Metabolischen Syndroms eingesetzt werden.

Außerdem können die erfindungsgemäßen Verbindungen zur Behandlung und/oder Prophylaxe von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, Gangren, CREST-Syndrom, Erythematose, Onychomykose, rheumatischen Erkrankungen sowie zur Förderung der Wundheilung verwendet werden.

Weiterhin eignen sich die erfindungsgemäßen Verbindungen zur Behandlung urologischer Erkrankungen wie beispielsweise benignes Prostata-Syndrom (BPS), benigne Prostata-Hyperplasie (BPH), benigne Prostata Vergrösserung (BPE), Blasenentleerungsstörung (BOO), untere Harnwegssyndrome (LUTS, einschließlich Felines Urologisches Syndrom (FUS)), Erkrankungen des Urogenital-Systems einschliesslich neurogene überaktive Blase (OAB) und (IC), Inkontinenz (UI) wie beispielsweise Misch-, Drang-, Stress-, oder Überlauf-Inkontinenz (MUI, UUI, SUI, OUI), Beckenschmerzen, benigne und maligne Erkrankungen der Organe des männlichen und weiblichen Urogenital-Systems.

Weiterhin eignen sich die erfindungsgemäßen Verbindungen zur Behandlung und/oder Prophylaxe von Nierenerkrankungen, insbesondere von aktuer und chronischer Niereninsuffizienz, sowie von akutem und chronischem Nierenversagen. Im Sinne der vorliegenden Erfindung umfasst der Begriff Niereninsuffizienz sowohl akute als auch chronische Erscheinungsformen der Niereninsuffizienz, wie auch zugrundeliegende oder verwandte Nierenerkrankungen wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunlogische Nierenerkrankungen wie Nierentransplantatabstoßung, Immunkomplex-induzierte Nierenerkrankungen, durch toxische Substanzen induzierte Nephropathie, Kontrastmittel-induzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prophylaxe von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weiterhin eignen sich die erfindungsgemäßen Verbindungen auch zur Behandlung und/oder Prophylaxe von asthmatischen Erkrankungen, pulmonaler arterieller Hypertonie (PAH) und anderen Formen der pulmonalen Hypertonie (PH), der chronisch-obstruktive Lungenerkrankung (COPD), des akuten Atemwegssyndrom (ARDS), der akuten Lungenschädigung (ALI), der alpha-1-Antitrypsin-Defizienz (AATD), der Lungenfibrose, des Lungenemphysem (z.B. durch Zigarettenrauch induziertes Lungenemphysem) und der zystischen Fibrose (CF).

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Demyelinisation, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung und/oder Prophylaxe von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen und Tinnitus eingesetzt werden.

Zudem besitzen die erfindungsgemäßen Verbindungen antiinflammatorische Wirkung und können daher als entzündungshemmende Mittel zur Behandlung und/oder Prophylaxe von Sepsis (SIRS), multiplem Organversagen (MODS, MOF), entzündlichen Erkrankungen der Niere, chronischen Darmentzündungen (IBD, Crohn's Disease, UC), Pankreatitis, Peritonitis, rheumatoiden Erkrankungen, entzündlichen Hauterkrankungen sowie entzündlichen Augenerkrankungen eingesetzt werden.

Des weiteren können die erfindungsgemäßen Verbindungen ebenfalls zur Behandlung und/ oder Prophylaxe von Autoimmunerkrankungen eingesetzt werden.

Weiterhin sind die erfindungsgemäßen Verbindungen zur Behandlung und/ oder Prophylaxe fibrotischer Erkrankungen der inneren Organe, wie beispielsweise der Lunge, des Herzens, der Niere, des Knochenmarks und insbesondere der Leber, sowie dermatologischer Fibrosen und fibrotischer Erkrankungen des Auges, geeignet. Im Sinne der vorliegenden Erfindungen umfasst der Begriff fibrotischer Erkrankungen insbesondere die folgenden Begriffe Leberfibrose, Leberzirrhose, Lungenfibrose, Endomyocardfibrose, Nephropathie, Glomerulonephritis, interstitielle Nierenfibrose, fibrotische Schäden in Folge von Diabetes, Knochenmarksfibrose und ähnliche fibrotische Erkrankungen, Sklerodermie, Morphaea, Keloide, hypertrophe Narbenbildung (auch nach chirurgischen Eingriffen), Naevi, diabetische Retinopathie und proliferative Vitroretinopathie.

Weiterhin eignen sich die erfindungsgemäßen Verbindungen zur Bekämpfung postoperativer Narbenbildung, z.B. in Folge von Glaukom-Operationen.

Die erfindungsgemäßen Verbindungen können ebenfalls kosmetisch bei alternder und verhornender Haut eingesetzt werden.

Außerdem sind die erfindungsgemäßen Verbindungen zur Behandlung und/ oder Prophylaxe von Hepatitis, Neoplasma, Osteoporose, Glaukom und Gastroparese geeignet.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung sind die erfindungsgemäßen Verbindungen zur Verwendung in einem Verfahren zur Bhandlung und/ oder Prophylaxe von Herzinsuffizienz, Angina pectoris, Hypertonie, pulmonaler Hypertonie, Ischämien, Gefäßerkrankungen, thromboembolischen Erkrankungen und Arteriosklerose.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Dabigatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Diuretikum, wie beispielhaft und vorzugsweise Furosemid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Dalcetrapib, BAY 60-5521, Anacetrapib oder CETP-vaccine (CETi-1), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- aq.: wässrige Lösung
- ber.: Berechnet
- DCI: direkte chemische Ionisation (bei MS)
- DMF: Dimethylformamid
- DMSO: Dimethylsulfoxid
- d. Th.: der Theorie (bei Ausbeute)
- eq.: Äquivalent(e)
- ESI: Elektrospray-Ionisation (bei MS)
- Et: Ethyl
- gef.: Gefunden
- h: Stunde(n)
- HPLC: Hochdruck-, Hochleistungsflüssigchromatographie
- HRMS: hochaufgelöste Massenspektrometrie
- konz.: konzentriert
- LC/MS: Flüssigchromatographie-gekoppelte Massenspektrometrie
- LiHMDS: Lithiumhexamethyldisilazid
- Me: Methyl
- min: Minute(n)
- MS: Massenspektrometrie
- NMR: Kernresonanzspektrometrie
- Pd₂dba₃: Tris-(dibenzylidenaceton)-dipalladium
- Ph: Phenyl
- RT: Raumtemperatur
- Rₜ: Retentionszeit (bei HPLC)
- THF: Tetrahydrofuran
- UV: Ultraviolett-Spektrometrie
- v/v: Volumen zu Volumen-Verhältnis (einer Lösung)
- XPHOS: Dicyclohexyl-(2',4',6'-triisopropylbiphenyl-2-yl)-phosphin

### LC/MS-Methoden:

### Methode 1 (LC-MS):

Instrument: Waters ACQUITY SQD UPLC System; Säule: Waters Acquity UPLC HSS T3 1,8µ 50 x 1mm; Eluent A: 1 1 Wasser + 0.25 ml 99%ige Ameisensäure , Eluent B: 11 Acetonitril + 0.25 ml 99%ige Ameisensäure; Gradient: 0.0 min 90% A → 1.2 min 5% A → 2.0 min 5% A; Ofen: 50°C; Fluss: 0.40 ml/min; UV-Detektion: 210 - 400 nm.

### Methode 2 (LC-MS):

Instrument: Micromass Quattro Micro MS mit HPLC Agilent Serie 1100; Säule: Thermo Hypersil GOLD 3µ 20 mm x 4 mm; Eluent A: 1 1 Wasser + 0.5 ml 50%-ige Ameisensäure, Eluent B: 11 Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Ausgangsverbindungen und Intermediate:

### Beispiel 1A

### 3,5-Difluorpyridin-2-carbonylchlorid

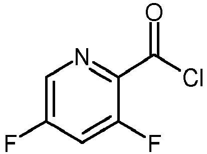

Eine Suspension von 5.00 g (31.4 mmol) 3,5-Difluorpyridin-2-carbonsäure in Thionylchlorid (21 mL) wurde 5 h zum Rückfluss erhitzt. Die Lösung wurde eingeengt, der Rückstand zweimal in wenig Toluol aufgenommen und wieder eingeengt. Man erhielt 3.80 g eines Feststoffs., der ohne weitere Reinigung direkt weiter umgesetzt wurde.

### Beispiel 2A

### Methyl-3-(3,5-difluorpyridin-2-yl)-2-(2-fluorphenyl)-3-oxopropanoat

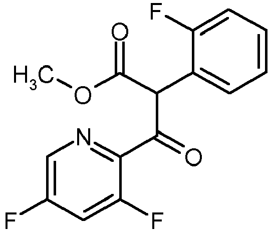

In THF (30 mL) wurden unter Argon 21.4 mL (21.4 mmol) Lithiumhexamethyldisilazid (1.0 M in THF) vorgelegt und bei -78°C eine Lösung von 3.00 g (17.8 mmol) 2-Fluorphenylessigsäuremethylester in THF (15 mL) zugetropft. Die Reaktionsmischung wurde 1 h bei -78°C gerührt und anschließend eine Lösung von 3.80 g (21.4 mmol) der Verbindung aus Beispiel 1A in THF (15 mL) zugetropft. Die Lösung wurde 1h bei -78°C gerührt, danach auf RT gebracht und portionsweise mit gesättigter wässriger Ammoniumchlorid-Lösung versetzt. Die Mischung wurde mit Wasser verdünnt und zweimal mit Ethylacetat extrahiert. Die vereinigten organischen Phasen wurden über Natriumsulfat getrocknet, filtriert und eingeengt. Der Rückstand wurde mit MTBE verrührt, der Feststoff abfiltriert und das Filtrat eingeengt. Kieselgelchromatographie (Laufmittel: Cyclohexan-Ethylacetat 30:1, 20:1) des Rückstands lieferte 3.66 g (87%-ige Reinheit, 57 % d. Th.) der Titelverbindung. Das Rohprodukt wurde ohne weitere Reinigung umgesetzt.

LC-MS (Methode 1): Rₜ = 1.05 min; MS (ESIpos): m/z = 310 (M+H)⁺

¹H-NMR (400 MHz, DMSO-d₆): δ = 3.66 (s, 3H), 6.25 (s, 1H), 7.20 - 7.28 (m, 4H), 7.31 - 7.38 (m, 1H), 8.15 - 8.23 (m, 1H), 8.68 - 8.71 (m, 1H).

### Beispiel 3A

### 1-(3,5-Difluorpyridin-2-yl)-2-(2-fluorphenyl)ethanon

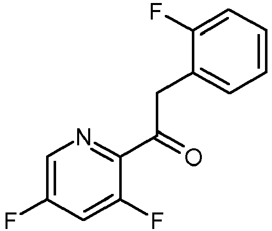

In DMSO (37 mL) wurden 11.65 g (37.67 mmol) der Verbindung aus Beispiel 2A vorgelegt. Anschließend wurden 2.42 g (41.44 mmol) Natriumchlorid sowie Wasser (7 mL) zugefügt und die Mischung 30 min bei 150°C in der Mikrowelle gerührt. Die Reaktionsmischung wurde mit Ethylacetat verdünnt, die organische Phase dreimal mit Wasser sowie einmal mit gesättigter wässriger Natriumchlorid-Lösung gewaschen, über Natriumsulfat getrocknet, filtriert und eingeengt. Man erhielt 9.07 g (89%ig, 85 % d. Th.) der gewünschten Verbindung als Feststoff, der ohne weitere Reinigung umgesetzt wurde.

LC-MS (Methode 1): Rₜ = 1.05 min; MS (ESIpos): m/z = 252 (M+H)⁺

¹H-NMR (400 MHz, DMSO-d₆): δ = 4.53 (s, 2H), 7.15 - 7.22 (m, 2H), 7.30 - 7.37 (m, 2H), 8.11 - 8.18 (m, 1H), 8.70 - 8.72 (m, 1H).

### Beispiel 4A

### 6-Fluor-3-(2-fluorbenzyl)-1H-pyrazolo[4,3-b]pyridin

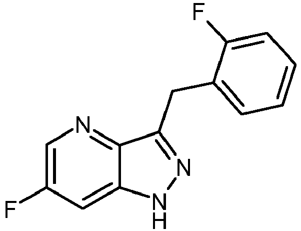

In Pyridin (84 mL) wurden 9.07 g (32.4 mmol) der Verbindung aus Beispiel 3A vorgelegt. Anschließend wurden 8.10 g (162 mmol) Hydrazinhydrat sowie 19.8 mg (0.162 mmol) 4-Dimethylaminopyridin zugefügt und die Mischung 30 min zum Rückfluss erhitzt. Die Reaktionsmischung wurde bei RT mit Ethylacetat verdünnt und viermal mit 10%-iger wässriger Zitronensäure-Lösung gewaschen. Die organische Phase wurde anschließend mit gesättigter wässriger Natriumchlorid-Lösung gewaschen, über Natriumsulfat getrocknet, filtriert und eingeengt. Der Rückstand wurde mit MTBE versetzt und der Feststoff abfiltriert. Dieser wurde im Hochvakuum getrocknet und lieferte 1.79 g (79%ig, 18% d. Th.) der Titelverbindung. Das Filtrat wurde eingeengt und lieferte weitere 4.86 g (61%ig, 37% d. Th.) der Titelverbindung. Die beiden Fraktionen wurden vereinigt und ohne weitere Reinigung umgesetzt.

LC-MS (Methode 2): Rₜ = 1.87 min; MS (ESIpos): m/z = 246 (M+H)⁺

¹H-NMR (400 MHz, DMSO-d₆): δ = 4.33 (s, 2H), 7.06 - 7.12 (m, 1H), 7.12 - 7.19 (m, 1H), 7.22 - 7.29 (m, 1H), 7.29 - 7.35 (m, 1H), 7.87 (dd, 1H), 7.84 - 7.89 (m, 1H), 8.48 - 8.51 (br. s, 1H).

### Beispiel 5A

### 2-[6-Fluor-3-(2-fluorbenzyl)-1H-pyrazolo[4,3-b]pyridin-1-yl]-5-nitropyrimidin-4,6-diamin

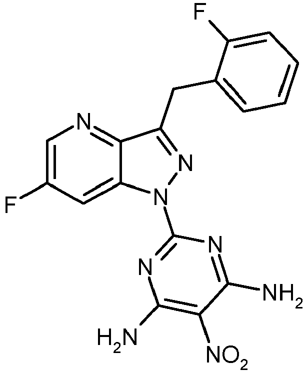

In DMF (3.5 mL) wurden 156 mg (ca. 66% ige Reinheit, 0.636 mmol) der Verbindung aus Beispiel 4A gelöst, anschließend 28 mg (0.70 mmol) Natriumhydrid (60%ig in Mineralöl) zugefügt und die Mischung 2 h bei RT gerührt. Danach wurden 115 mg (0.604 mmol) 2-Chlor-5-nitropyrimidin-4,6-diamin (Synthese: Helvetica Chimica Acta (1951), 34, 835-40) zugefügt und die Reaktionsmischung für 1 h bei 80°C nachgerührt. Nach Abkühlen auf RT wurde die Mischung auf Wasser gegeben. Die so erhaltene Suspension wurde filtriert, der Feststoff mehrfach mit Wasser gewaschen und anschließend im Hochvakuum getrocknet. Man erhielt 196 mg (77 % d. Th.) der Titelverbindung als Feststoff.

### LC-MS (Methode 2): Rₜ = 2.13 min; MS (ESIpos): m/z = 399 (M+H)⁺

### Beispiel 6A

### 2-[6-Fluor-3-(2-fluorbenzyl)-1H-pyrazolo[4,3-b]pyridin-1-yl]pyrimidin-4,5,6-triamin

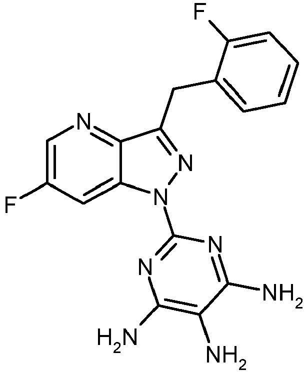

In Pyridin (22 mL) wurden 196 mg (0.492 mmol) der Verbindung aus Beispiel 5A vorgelegt, anschließend 74 mg Palladium auf Kohle (10%ig w/w) zugefügt und die Mischung über Nacht bei Wasserstoff-Normaldruck hydriert. Anschließend wurde die Reaktionsmischung durch Kieselgur filtriert, mit Ethanol nachgewaschen und das Filtrat eingeengt. Der Rückstand wurde mit Ethanol bei 50°C verrührt, der Feststoff abfiltriert und im Hochvakuum getrocknet. Man erhielt 107 mg (58 % d. Th.) der Titelverbindung.

LC-MS (Methode 1): Rₜ = 0.85 min; MS (ESIpos): m/z = 369 (M+H)⁺

¹H-NMR (400 MHz, DMSO-d₆): δ = 3.76 (s, 2H), 4.39 (s, 2H), 6.12 (s, 4H), 7.07 - 7.14 (m, 1H), 7.14 - 7.21 (m, 1H), 7.23 - 7.31 (m, 1H), 7.31 - 7.37 (m, 1H), 8.58 - 8.62 (m, 1H), 8.94 (dd, 1H).

### Ausführungsbeispiele:

### Beispiel 1

### Methyl-{4,6-diamino-2-[6-fluor-3-(2-fluorbenzyl)-1H-pyrazolo[4,3-b]pyridin-1-yl]pyrimidin-5-yl}carbamat

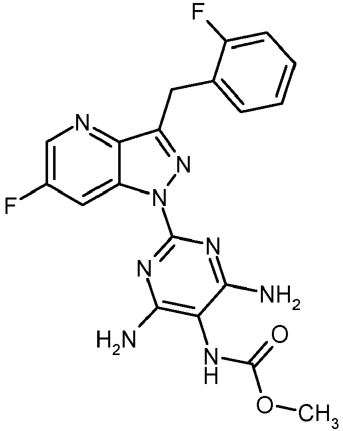

In Pyridin (55 mL) wurden unter Argon 1.00 g (2.72 mmol) der Verbindung aus Beispiel 6A vorgelegt, die Mischung auf 0°C abgekühlt und anschließend 228 µL (2.72 mmol) Chlorameisensäuremethylester (Lösung in 10 mL Dichlormethan) zugetropft. Die Reaktionsmischung wurde über Nacht bei RT weiter gerührt und anschließend eingeengt. Der Rückstand wurde mit Ethanol ausgerührt, der Feststoff abfiltriert und bei 50°C im Hochvakuum getrocknet. Man erhielt 873 mg (75 % d. Th.) der Titelverbindung als beigefarbenen Feststoff. Das Filtrat wurde erneut eingeengt, und die Reinigung mittels präparativer RP-HPLC (Acetonitril:Wasser (+0.1% Ameisensäure) - Gradient) des Rückstands lieferte weitere 180 mg (16 % d. Th.) der Titelverbindung.

LC-MS (Methode 1): Rₜ = 0.88 min; MS (ESIpos): m/z = 427 (M+H)⁺

¹H-NMR (400 MHz, DMSO-d₆, Rotamerengemisch): δ [ppm]= 3.48 - 3.67 (m, 3H), 4.40 (s, 2H), 6.45 (br. s, 4H), 7.08 - 7.14 (m, 1H), 7.15 - 7.21 (m, 1H), 7.24 - 7.31 (m, 1H), 7.31 - 7.37 (m, 1H), 7.60 (br. s, 0.2H), 7.90 (br. s, 0.8H), 8.61 - 8.65 (m, 1H), 9.04 (dd, 1H).

### Beispiel 2

### Ethyl-{4,6-diamino-2-[6-fluor-3-(2-fluorbenzyl)-1H-pyrazolo[4,3-b]pyridin-1-yl]pyrimidin-5-yl}carbamat

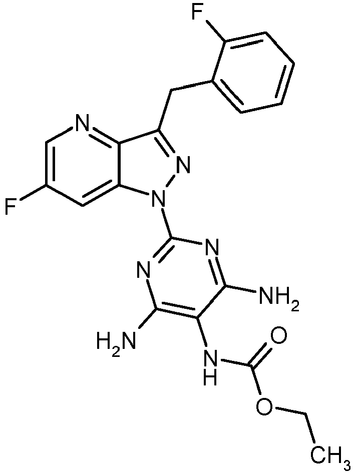

In Pyridin (10 mL) wurden unter Argon 300 mg (0.814 mmol) der Verbindung aus Beispiel 6A vorgelegt, die Mischung auf 0°C abgekühlt und anschließend 78 µL (0.814 mmol) Chlorameisensäureethylester zugetropft. Die Reaktionsmischung wurde 1h bei 0°C nachgerührt und anschließend eingeengt. Der Rückstand wurde mittels präparativer RP-HPLC (Acetonitril:Wasser (+0.1% Ameisensäure) - Gradient) getrennt und die Produktfraktionen eingeengt. Man erhielt 77 mg (21 % d. Th.) der Titelverbindung.

LC-MS (Methode 1): Rₜ = 0.92 min; MS (ESIpos): m/z = 441 (M+H)⁺.

¹H-NMR (400 MHz, DMSO-d₆, Rotamerengemisch): δ = 1.05 - 1.29 (m, 3H), 3.99 - 4.11 (m, 2H), 4.40 (s, 2H), 6.44 (br. s, 4H), 7.08 - 7.13 (m, 1H), 7.15 - 7.21 (m, 1H), 7.24 - 7.31 (m, 1H), 7.31 - 7.37 (m, 1H), 7.55 (br. s, 0.25H), 7.88 (br. s, 0.75H), 8.61 - 8.65 (m, 1H), 9.02 - 9.08 (m, 1H).

### Beispiel 3

### Isopropyl-{4,6-diamino-2-[6-f[uor-3-(2-fluorbenzyl)-1H-pyrazolo[4,3-b]pyridin-1-yl]pyrimidin-5-yl}carbamat

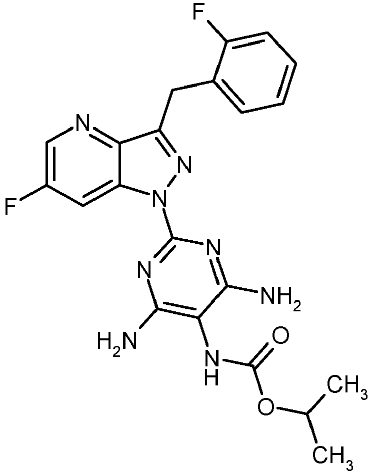

Die Substanz wurde in Analogie zur Synthese von Beispiel 2 aus Beispiel 6A hergestellt.

Ausbeute: 88 mg (24 % d. Th.)

LC-MS (Methode 1): Rₜ = 0.97 min; MS (ESIpos): m/z = 455 (M+H)⁺.

¹H-NMR (400 MHz, DMSO-d₆, Rotamerengemisch): δ [ppm]= 1.07 - 1.29 (m, 6H), 4.40 (s, 2H), 4.77 - 4.86 (m, 1H), 6.39 (br. s, 4H), 7.08 - 7.13 (m, 1H), 7.15 - 7.21 (m, 1H), 7.24 - 7.31 (m, 1H), 7.31 - 7.37 (m, 1H), 7.48 (br. s, 0.25H), 7.81 (br. s, 0.75H), 8.61 - 8.65 (m, 1H), 9.05 (dd, 1H).

### Beispiel 4

### Cyclobutyl-{4,6-diamino-2-[6-fluor-3-(2-fluorbenzyl)-1H-pyrazolo[4,3-b]pyridin-1-yl]pyrimidin-5-yl}carbamat

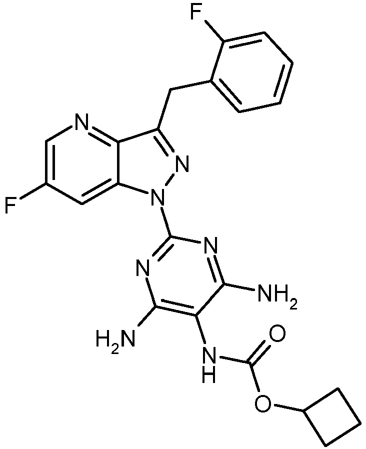

In Dichlormethan (4.1 mL) wurden unter Argon 64 µL (0.81 mmol) Cyclobutylalkohol vorgelegt, danach mit 161 mg (0.54 mmol) Bis(trichlormethyl)carbonat versetzt und die Mischung auf 0°C abgekühlt. Anschließend wurden 53 µL (0.65 mmol) Pyridin zugetropft und die Reaktionsmischung 30 min bei 0°C nachgerührt. Danach wurden 200 mg (0.54 mmol) der Verbindung aus Beispiel 6A zugefügt und die Mischung mit 1.4 mL Pyridin verdünnt. Die Reaktionsmischung wurde über Nacht bei RT gerührt, anschließend mit gesättigter wässriger Natriumhydrogencarbonatlösung versetzt und zweimal mit Ethylacetat extrahiert. Die gesammelten organischen Phasen wurden über Natriumsulfat getrocknet, filtriert und eingeengt. Der Rückstand wurde mittels präparativer RP-HPLC (Acetonitril:Wasser (+0.1% Ameisensäure) - Gradient) gereinigt und die Produktfraktionen eingeengt.

Ausbeute: 194 mg (77 % d. Th.)

LC-MS (Methode 1): Rₜ = 0.99 min; MS (ESIpos): m/z = 467 (M+H)⁺.

¹H-NMR (400 MHz, DMSO-d₆, Rotamerengemisch):□δ [ppm] = 1.47 - 1.64 (m, 1H), 1.66 - 1.80 (m, 1H), 1.83 - 2.17 (m, 0.5H), 2.20 - 2.33 (m, 1.5H), 4.40 (s, 2H), 4.80 - 4.93 (m, 1H), 6.42 (br. s, 4H), 7.07 - 7.14 (m, 1H), 7.14 - 7.22 (m, 1H), 7.23 - 7.31 (m, 1H), 7.31 - 7.38 (m, 1H), 7.47 - 7.58 (m, 0.25H), 7.82 - 7.92 (m, 0.75H), 8.59 - 8.65 (m, 1H), 9.00 - 9.08 (m, 1H).

### Beispiel 5

### Cyclopropylmethyl-{4,6-diamino-2-[6-fluor-3-(2-fluorbenzyl)-1H-pyrazolo[4,3-b]pyridin-1-yl]pyrimidin-5-yl}carbamat

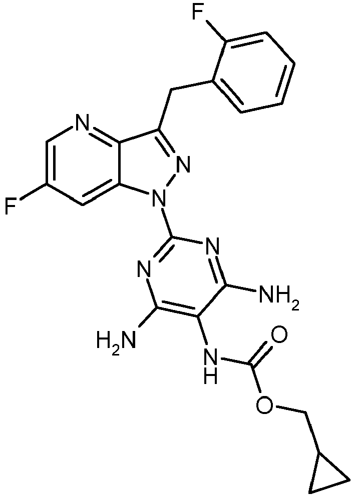

Die Substanz wurde in Analogie zur Synthese von Beispiel 4 unter Verwendung von Cyclopropylmethanol hergestellt.

Ausbeute: 191 mg (41 % d. Th.)

LC-MS (Methode 1): Rₜ = 0.97 min; MS (ESIpos): m/z = 467 (M+H)⁺.

¹H-NMR (400 MHz, DMSO-d₆, Rotamerengemisch):δ [ppm] = 0.18 - 0.36 (m, 2H), 0.37 - 0.59 (m, 2H), 1.08 - 1.19 (m, 1H), 3.82 - 3.88 (m, 2H), 4.40 (s, 2H), 6.42 (br. s, 4H), 7.08 - 7.14 (m, 1H), 7.14 - 7.21 (m, 1H), 7.23 - 7.31 (m, 1H), 7.31 - 7.37 (m, 1H), 7.56 (br. s, 0.25H), 7.94 (br. s, 0.75H), 8.60 - 8.65 (m, 1H), 9.01 - 9.08 (m, 1H).

### Beispiel 6

### Oxetan-3-yl-{4,6-diamino-2-[6-fluor-3-(2-fluorbenzyl)-1H-pyrazolo[4,3-b]pyridin-1-yl]pyrimidin-5-yl}carbamat

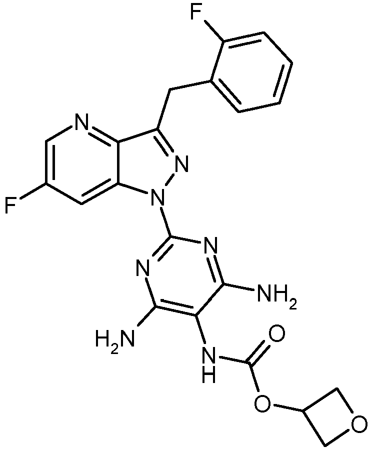

In Dichlormethan (6.2 mL) wurden unter Argon 60.4µl (0.98 mmol) 3-Hydroxyoxethan vorgelegt, danach mit 0.5 eq Bis(trichlormethyl)carbonat versetzt und die Mischung auf 0°C abgekühlt. Anschließend wurde 79 µl (0.98 mmol) Pyridin zugetropft, die Reaktionsmischung auf RT gebracht und 1 h nachgerührt. Danach wurde erneut auf 0°C abgekühlt und schließlich eine Lösung von 334 mg (0.815 mmol) der Verbindung aus Beispiel 6A in Pyridin (2 mL) zugefügt. Die Reaktionsmischung wurde über Nacht bei RT gerührt, anschließend mit gesättigter wässriger Natriumhydrogencarbonatlösung versetzt und zweimal mit Ethylacetat extrahiert. Die gesammelten organischen Phasen wurden über Natriumsulfat getrocknet, filtriert und eingeengt. Der Rückstand wurde mittels präparativer RP-HPLC (Acetonitril:Wasser (+0.1% Ameisensäure) - Gradient) aufgetrennt und die Produktfraktionen eingeengt. Das so erhaltene Rohprodukt wurde an Kieselgel (Dichlormethan:Methanol 100:1, 30:1) chromatographiert und abschließend mittels präparativer RP-HPLC (Acetonitril:Wasser (+0.1% Ameisensäure) - Gradient) erneut gereinigt.

Ausbeute: 19 mg (5 % d. Th.)

LC-MS (Methode 1): Rₜ = 0.87 min; MS (ESIpos): m/z = 469 (M+H)⁺.

¹H-NMR (400 MHz, DMSO-d₆, Rotamerengemisch): δ [ppm] = 4.40 (s, 1H), 4.43 - 4.48 (m, 0.5H), 4.61 - 4.66 (m, 1.5H), 4.67 - 4.73 (m, 0.5H), 4.75 - 4.82 (m, 1.5H), 5.26 - 5.38 (m, 1H), 6.45 - 6.63 (m, 4H), 7.07 - 7.14 (m, 1H), 7.15 - 7.22 (m, 1H), 7.24 - 7.31 (m, 1H), 7.31 - 7.37 (m, 1H), 7.78 - 7.82 (m, 0.25H), 8.12 - 8.18 (m, 0.75H), 8.61 - 8.66 (m, 1H), 9.01 - 9.09 (m, 1H).

### Beispiel 7

### Methyl-14,6-diamino-2-[6-fluor-3-(2-fluorbenzyl)-1H-pyrazolo[4,3-b]pyridin-1-yl]pyrimidin-5-yl}methylcarbamat

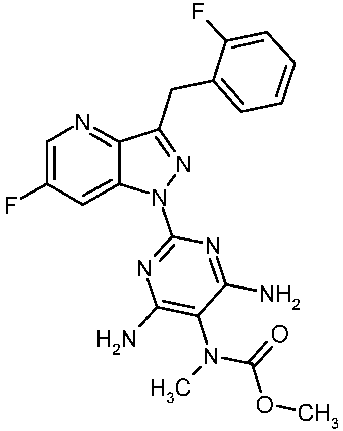

In THF (3.3 mL) wurden unter Argon 50 mg (0.12 mmol) der Verbindung aus Beispiel 1 vorgelegt, die Mischung auf -20°C abgekühlt und anschließend 0.13 mL (0.13 mmol) Bis(trimethylsilyl)natriumamid (1.0 M in THF) zugetropft. Es wurde 30 min bei -20°C nachgerührt und anschließend 6.6 µL (0.11 mmol) Iodmethan zugetropft. Die Reaktionsmischung wurde weitere 10 min bei -20°C sowie über Nacht bei RT nachgerührt. Danach wurden weitere 1.0 eq Iodmethan als Lösung in 1.0 mL THF zugetropft und erneut über Nacht bei RT gerührt. Die Reaktionsmischung wurde mit Ethylacetat verdünnt und zweimal mit gesättigter wässriger Natriumhydrogencarbonatlösung gewaschen. Die organische Phase wurde über Magnesiumsulfat getrocknet, filtriert und eingeengt. Der Rückstand wurde mittels präparativer RP-HPLC (Acetonitril:Wasser (+0.1% Ameisensäure) - Gradient) aufgetrennt und die Produktfraktionen eingeengt. Man erhielt 13 mg (25 % d. Th.) der Titelverbindung als Feststoff.

LC-MS (Methode 1): Rₜ = 0.95 min; MS (ESIpos): m/z = 441 (M+H)⁺.

¹H-NMR (400 MHz, DMSO-d₆, Rotamerengemisch): δ [ppm]= 2.99 (s, 3H), 3.53 (s, 2.1H), 3.65 (s, 0.9H), 4.40 (s, 2H), 6.63 - 6.70 (m, 4H), 7.08 - 7.13 (m, 1H), 7.15 - 7.21 (m, 1H), 7.23 - 7.36 (m, 2H), 8.61 - 8.65 (m, 1H), 9.04 (dd, 1H).

### Beispiel 8

### Ethyl-{4,6-diamino-2-[6-fluor-3-(2-fluorbenzyl)-1H-pyrazolo[4,3-b]pyridin-1-yl]pyrimidin-5-yl} methylcarbamat

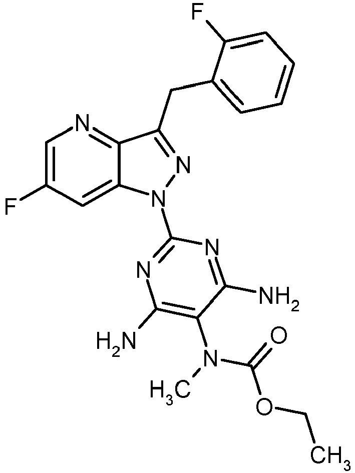

Die Verbindung wurde in Analogie zur Synthese von Beispiel 7 unter Verwendung von Iodmethan und der Verbindung aus Beispiel 2 hergestellt. Man erhielt 18 mg (38 % d. Th.) der Titelverbindung als Feststoff.

LC-MS (Methode 1): Rₜ = 0.99 min; MS (ESIpos): m/z = 455 (M+H)⁺.

¹H-NMR (400 MHz, DMSO-d₆, Rotamerengemisch): δ [ppm]= 1.09 (t, 2H), 1.27 (t, 1H), 2.99 (s, 3H), 4.09 (q, 1.5H), 4.07 (q, 0.5H), 4.40 (s, 2H), 6.59 - 6.69 (m, 4H), 7.08 - 7.13 (m, 1H), 7.15 - 7.21 (m, 1H), 7.24 - 7.29 (m, 1H), 7.30 - 7.37 (m, 1H), 8.61 - 8.64 (m, 1H), 9.01 - 9.07 (m, 1H).

### Beispiel 9

### Isopropyl-{4,6-diamino-2-[6-fluor-3-(2-fluorbenzyl)-1H-pyrazolo[4,3-b]pyridin-1-yl]pyrimidin-5-yl}methylcarbamat

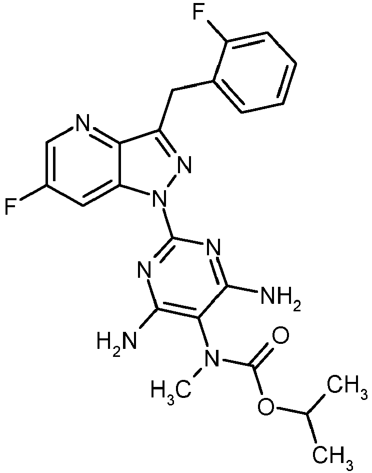

Die Verbindung wurde in Analogie zur Synthese von Beispiel 7 unter Verwendung von Iodmethan und der Verbindung aus Beispiel 3 hergestellt. Man erhielt 28 mg (48 % d. Th.) der Titelverbindung als Feststoff.

LC-MS (Methode 1): Rₜ = 0.99 min; MS (ESIpos): m/z = 469 (M+H)⁺.

¹H-NMR (400 MHz, DMSO-d₆, Rotamerengemisch): δ [ppm]= 1.10 (d, 4.5H), 1.27 (d, 1.5H), 2.98 (s, 3H), 4.39 (s, 2H), 4.75 - 4.84 (m, 1H), 6.55 - 6.66 (m, 4H), 7.08 - 7.14 (m, 1H), 7.15 - 7.21 (m, 1H), 7.24 - 7.37 (m, 2H), 8.61 - 8.65 (m, 1H), 9.02 - 9.08 (m, 1H).

### Beispiel 10

### Methyl-{4,6-diamino-2-[6-fluor-3-(2-fluorbenzyl)-1H-pyrazolo[4,3-b]pyridin-1-yl]pyrimidin-5-yl} ethylcarbamat

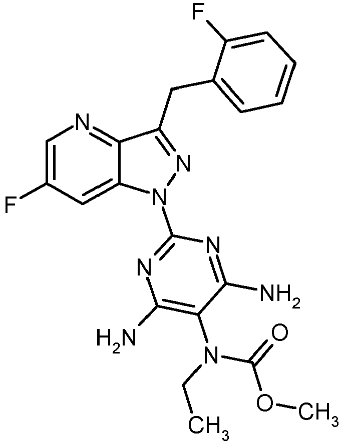

In THF (9.5 mL) wurden unter Argon 142 mg (0.33 mmol) der Verbindung aus Beispiel 1 vorgelegt, die Mischung auf 0°C abgekühlt und anschließend 0.33 mL (0.33 mmol) Bis(trimethylsilyl)natriumamid (1.0 M in THF) zugetropft. Es wurde 30 min bei 0°C nachgerührt und anschließend 35 µL (0.43 mmol) Iodethan zugetropft. Die Reaktionsmischung wurde über Nacht bei RT nachgerührt. Die Reaktionsmischung wurde mit Ethylacetat verdünnt und zweimal mit gesättigter wässriger Natriumhydrogencarbonatlösung gewaschen. Die organische Phase wurde über Magnesiumsulfat getrocknet, filtriert und eingeengt. Der Rückstand wurde an Kieselgel chromatographiert (Laufmittel Dichlormethan:Methanol 60:1) und die Produktfraktionen eingeengt. Man erhielt 89 mg (59 % d. Th.) der Titelverbindung als Feststoff.

LC-MS (Methode 1): Rₜ = 1.01 min; MS (ESIpos): m/z = 455 (M+H)⁺.

¹H-NMR (400 MHz, DMSO-d₆, Rotamerengemisch): δ [ppm]= 1.06 (t, 3H), 3.43 - 3.53 (m, 2H), 3.54 (s, 2H), 3.67 (s, 1H), 4.40 (s, 2H), 6.54 - 6.63 (m, 4H), 7.08 - 7.13 (m, 1H), 7.15 - 7.21 (m, 1H), 7.24 - 7.36 (m, 2H), 8.60 - 8.64 (m, 1H), 9.05 (dd, 1H).

### Beispiel 11

### Ethyl-{4,6-diamino-2-[6-fluor-3-(2-fluorbenzyl)-1H-pyrazolo[4,3-b]pyridin-1-yl]pyrimidin-5-yl}ethylcarbamat

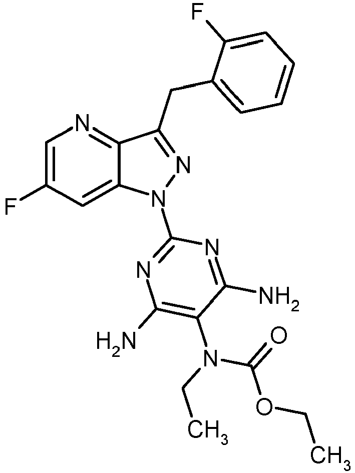

Die Verbindung wurde in Analogie zur Synthese von Beispiel 10 und Verwendung der Verbindung aus Beispiel 2 hergestellt. Die Verbindung wurde mittels präparativer RP-HPLC (Acetonitril:Wasser (+0.1% Ameisensäure) - Gradient) isoliert. Man erhielt 38 mg (35 % d. Th.) der Titelverbindung als Feststoff.

LC-MS (Methode 1): Rₜ = 1.05 min; MS (ESIpos): m/z = 469 (M+H)⁺.

¹H-NMR (400 MHz, DMSO-d₆, Rotamerengemisch): δ [ppm]= 1.02 - 1.14 (m, 5H), 1.27 (t, 1H), 3.41 - 3.54 (m, 2H), 3.98 - 4.14 (m, 2H), 4.40 (s, 2H), 6.49 - 6.60 (m, 4H), 7.08 - 7.13 (m, 1H), 7.15 - 7.21 (m, 1H), 7.23 - 7.37 (m, 2H), 8.61 - 8.64 (m, 1H), 9.03 - 9.09 (m, 1H).

### Beispiel 12

### Isopropyl-{4,6-diamino-2-[6-fluor-3-(2-fluorbenzyl)-1H-pyrazolo[4,3-b]pyridin-1-yl]pyrimidin-5-yl}ethylcarbamat

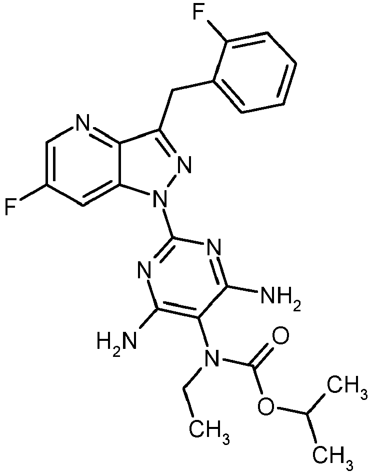

Die Verbindung wurde in Analogie zur Synthese von Beispiel 10 und Verwendung der Verbindung aus Beispiel 3 hergestellt. Man erhielt 64 mg (56 % d. Th.) der Titelverbindung als Feststoff.

LC-MS (Methode 1): Rₜ = 1.07 min; MS (ESIpos): m/z = 483 (M+H)⁺.

¹H-NMR (400 MHz, DMSO-d₆, Rotamerengemisch): δ [ppm]= 1.02 - 1.15 (m, 7.5H), 1.23 - 1.30 (m, 1.5H), 3.38 - 3.54 (m, 2H), 4.40 (s, 2H), 4.78 - 4.87 (m, 1H), 6.45 - 6.58 (m, 4H), 7.08 - 7.14 (m, 1H), 7.15 - 7.22 (m, 1H), 7.23 - 7.37 (m, 2H), 8.60 - 8.65 (m, 1H), 9.03 - 9.11 (m, 1H).

### Beispiel 13

### Methyl-{4,6-diamino-2-[6-fluor-3-(2-fluorbenzyl)-1H-pyrazolo[4,3-b]pyridin-1-yl]pyrimidin-5-yl} (2,2,2-trifluorethyl)carbamat

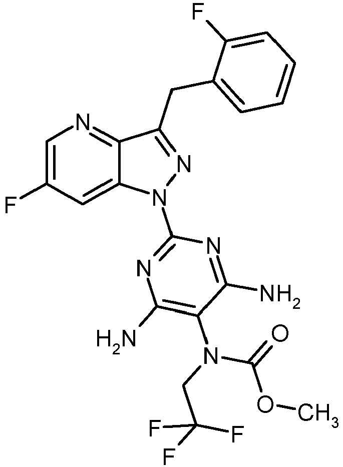

In THF (9.5 mL) wurden unter Argon 142 mg (0.33 mmol) der Verbindung aus Beispiel 1 vorgelegt, die Mischung auf 0°C abgekühlt und anschließend 13 mg (0.33 mmol) Natriumhydrid (60%ige Suspension in Mineralöl) zugegeben. Es wurde 30 min bei 0°C gerührt und anschließend 96 µL (0.67 mmol) 2,2,2-Trifluorethyltrichlormethansulfonat zugetropft. Die Reaktionsmischung wurde über Nacht bei RT gerührt. Anschließend wurde mit Ethylacetat verdünnt und die organische Phase zweimal mit gesättigter wässriger Natriumhydrogencarbonatlösung gewaschen. Die organische Phase wurde über Magnesiumsulfat getrocknet, filtriert und eingeengt. Der Rückstand wurde an Kieselgel chromatographiert (Laufmittel Dichlormethan:Methanol 60:1) und die Produktfraktionen eingeengt. Das so erhaltene Rohprodukt wurde mittels präparativer RP-HPLC (Acetonitril:Wasser (+0.1% Ameisensäure) - Gradient) nachgereinigt, anschließend in Acetonitril/Wasser suspendiert und schließlich durch Lyophilisation getrocknet. Man erhielt 68 mg (40 % d. Th.) der Titelverbindung als Feststoff.

LC-MS (Methode 1): Rₜ = 1.05 min; MS (ESIpos): m/z = 509 (M+H)⁺.

¹H-NMR (400 MHz, DMSO-d₆): δ [ppm]= 3.60 - 3.75 (m, 3H), 4.05 - 4.17 (m, 2H), 4.40 (s, 2H), 6.61 - 6.73 (m, 4H), 7.08 - 7.13 (m, 1H), 7.15 - 7.21 (m, 1H), 7.24 - 7.36 (m, 2H), 8.61 - 8.65 (m, 1H), 9.02 - 9.08 (m, 1H).

### Beispiel 14

### Isopropyl-{4,6-diamino-2-[6-fluor-3-(2-fluorbenzyl)-1H-pyrazolo[4,3-b]pyridin-1-yl]pyrimidin-5-yl} (2,2,2-trifluorethyl)carbamat

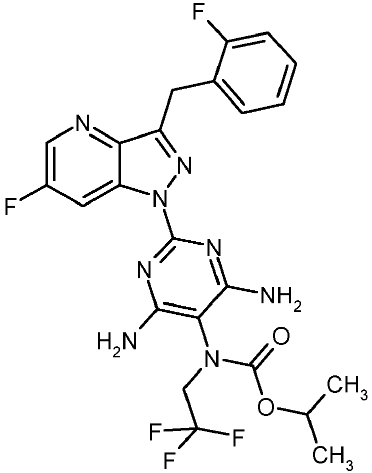

Die Herstellung erfolgte in Analogie zur Synthese von Beispiel 13 unter Verwendung der Verbindung aus Beispiel 3. Das Rohprodukt wurde mittels präparativer RP-HPLC (Acetonitril:Wasser (+0.1% Ameisensäure) - Gradient) und anschließende präparativer DC (Laufmittel Dichlormethan:Methanol 20:1) gereinigt. Man erhielt 38 mg (30 % d. Th.) der Titelverbindung als Feststoff.

LC-MS (Methode 1): Rₜ = 1.13 min; MS (ESIpos): m/z = 537 (M+H)⁺.

¹H-NMR (400 MHz, DMSO-d₆): δ [ppm]= 1.09 - 1.33 (m, 6H), 4.03 - 4.16 (m, 2H), 4.40 (s, 2H), 4.81 - 4.91 (m, 1H), 6.52 - 6.67 (br s, 4H), 7.08 - 7.13 (m, 1H), 7.14 - 7.21 (m, 1H), 7.24 - 7.37 (m, 2H), 8.61 - 8.66 (m, 1H), 9.03 - 9.10 (m, 1H).

### Beispiel 15

### Ethyl-{4,6-diamino-2-[6-fluor-3-(2-fluorbenzyl)-1H-pyrazolo[4,3-b]pyridin-1-yl]pyrimidin-5-yl} (2,2,2-trifluorethyl)carbamat

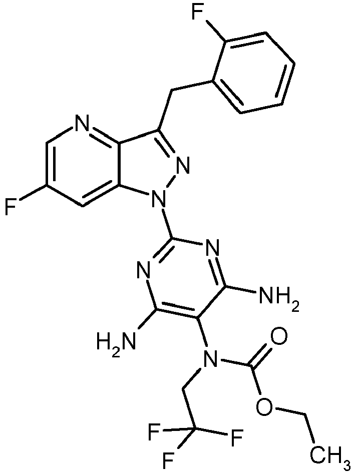

Die Herstellung erfolgte in Analogie zur Synthese von Beispiel 13 unter Verwendung der Verbindung aus Beispiel 2. Das Rohprodukt wurde mittels präparativer RP-HPLC (Acetonitril:Wasser (+0.1% Ameisensäure) - Gradient) gereinigt. Man erhielt 32 mg (26 % d. Th.) der Titelverbindung als Feststoff.

LC-MS (Methode 1): Rₜ = 1.11 min; MS (ESIpos): m/z = 523 (M+H)⁺.

¹H-NMR (400 MHz, DMSO-d₆): δ [ppm]= 1.08 - 1.32 (m, 3H), 4.03 - 4.20 (m, 4H), 4.40 (s, 2H), 6.64 (br s, 4H), 7.07 - 7.14 (m, 1H), 7.14 - 7.21 (m, 1H), 7.24 - 7.37 (m, 2H), 8.61 - 8.65 (m, 1H), 9.03 - 9.09 (m, 1H).

### B. Bewertung der pharmakologischen Wirksamkeit

Die pharmakologische Wirkung der erfindungsgemäßen Verbindungen kann in folgenden Assays gezeigt werden:

### B-1. Gefäßrelaxierende Wirkung in vitro

Kaninchen werden durch Nackenschlag betäubt und entblutet. Die Aorta wird entnommen, von anhaftendem Gewebe befreit, in 1.5 mm breite Ringe geteilt und einzeln unter einer Vorspannung in 5 ml-Organbäder mit 37°C warmer, Carbogen-begaster Krebs-Henseleit-Lösung folgender Zusammensetzung gebracht (jeweils mM): Natriumchlorid: 119; Kaliumchlorid: 4.8; Calciumchlorid-Dihydrat: 1; Magnesiumsulfat-Heptahydrat: 1.4; Kaliumdihydrogenphosphat: 1.2; Natriumhydrogencarbonat: 25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung zugesetzt und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50% zu reduzieren (IC₅₀-Wert). Das Standardapplikationsvolumen beträgt 5 µl, der DMSO-Anteil in der Badlösung entspricht 0.1%.

Repräsentative IC₅₀-Werte für die erfindungsgemäßen Verbindungen sind in der nachstehenden Tabelle (Tabelle 1) wiedergegeben:

**Tabelle 1:**

| Beispiel Nr. | IC₅₀ [nM] |
|---|---|
| 1 | 1380 |
| 2 | 763 |
| 7 | 194 |
| 8 | 286 |
| 13 | 178 |

### B-2. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

Repräsentative Werte (MEC = minimal effektive Konzentration) für die erfindungsgemäßen Verbindungen sind in der nachstehenden Tabelle (Tabelle 2) wiedergegeben:

**Tabelle 2:**

| Beispiel Nr. | MEC [µM] |
|---|---|
| 1 | 0.3 |
| 2 | 0.3 |
| 7 | 0.1 |
| 8 | 0.1 |
| 13 | 0.1 |

### B-3. Radiotelemetrische Blutdruckmessung an wachen, spontan hypertensiven Ratten

Für die im Folgenden beschriebene Blutdruckmessung an wachen Ratten wird ein im Handel erhältliches Telemetriesystem der Firma DATA SCIENCES INTERNATIONAL DSI, USA eingesetzt.

Das System besteht aus 3 Hauptkomponenten:
- Implantierbare Sender (Physiotel® Telemetrietransmitter)
- Empfänger (Physiotel® Receiver), die über einen Multiplexer (DSI Data Exchange Matrix) mit einem
- Datenakquisitionscomputer
verbunden sind.

Die Telemetrieanlage ermöglicht eine kontinuierliche Erfassung von Blutdruck Herzfrequenz und Körperbewegung an wachen Tieren in ihrem gewohnten Lebensraum.

### Tiermaterial

Die Untersuchungen werden an ausgewachsenen weiblichen spontan hypertensiven Ratten (SHR Okamoto) mit einem Körpergewicht von >200 g durchgeführt. SHR/NCrl von Okamoto Kyoto School of Medicine, 1963 wurden aus männlichen Wistar Kyoto Ratten mit stark erhöhtem Blutdruck und weiblichen mit leicht erhöhtem Blutdruck gekreuzt und in der F13 an die U.S. National Institutes of Health abgegeben.

Die Versuchstiere werden nach Senderimplantation einzeln in Makrolon - Käfigen Typ 3 gehalten. Sie haben freien Zugang zu Standardfutter und Wasser.

Der Tag - Nacht - Rhythmus im Versuchslabor wird per Raumbeleuchtung um 6:00 Uhr morgens und um 19:00 Uhr abends gewechselt.

### Senderimplantation

Die eingesetzten Telemetriesender TA11 PA - C40 werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobabital (Nembutal, Sanofi: 50mg/kg i.p. ) narkotisiert und an der Bauchseite weiträumig rasiert und desinfiziert. Nach Eröffnung des Bauchraumes entlang der Linea alba wird der flüssigkeitsgefüllte Meßkatheter des Systems oberhalb der Bifurcation nach cranial in die Aorta descendens eingesetzt und mit Gewebekleber (VetBonD TM, 3M) befestigt. Das Sendergehäuse wird intraperitoneal an der Bauchwandmuskulatur fixiert und die Wunde wird schichtweise verschlossen.

Postoperativ wird zur Infektionsprophylaxe ein Antibiotikum verabreicht (Tardomyocel COMP Bayer 1ml/kg s.c.)

### Substanzen und Lösungen

Wenn nicht anders beschrieben werden die zu untersuchenden Substanzen jeweils einer Gruppe von Tieren (n = 6 ) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5%-iger Tylose suspendiert.

Eine Lösungsmittel- behandelte Gruppe von Tieren wird als Kontrolle eingesetzt.

### Versuchsablauf

Die vorhandene Telemetrie - Meßeinrichtung ist für 24 Tiere konfiguriert. Jeder Versuch wird unter einer Versuchsnummer registiert (VJahr Monat Tag).

Den in der Anlage lebenden instrumentierten Ratten ist jeweils eine eigene Empfangsantenne zugeordnet (1010 Receiver, DSI).

Die implantierten Sender sind über einen eingebauten Magnetschalter von außen aktivierbar. Sie werden bei Versuchsvorlauf auf Sendung geschaltet. Die ausgestrahlten Signale können durch ein Datenakquisitionssystem (Dataquest TM A.R.T. for WINDOWS, DSI ) online erfasst und entsprechend aufgearbeitet werden. Die Ablage der Daten erfolgt jeweils in einem hierfür eröffneten Ordner der die Versuchsnummer trägt.

Im Standardablauf werden über je 10 Sekunden Dauer gemessen:
- Systolischer Blutdruck (SBP)
- Diastolischer Blutdruck (DBP)
- Arterieller Mitteldruck (MAP)
- Herzfrequenz (HR)
- Aktivität (ACT).

Die Messwerterfassung wird rechnergesteuert in 5 Minuten Abständen wiederholt. Die als Absolutwert erhobenen Quelldaten werden im Diagramm mit dem aktuell gemessenen Barometerdruck (Ambient Pressure Reference Monitor; APR-1) korrigiert und in Einzeldaten abgelegt. Weitere technische Details sind der umfangreichen Dokumentation der Herstellerfirma (DSI) zu entnehmen.

Wenn nicht anders beschrieben erfolgt die Verabreichung der Prüfsubstanzen am Versuchstag um 9.00 Uhr. Im Anschluss an die Applikation werden die oben beschriebenen Parameter 24 Stunden gemessen.

### Auswertung

Nach Versuchsende werden die erhobenen Einzeldaten mit der Analysis-Software (DATAQUEST TM A. R.T. TM ANALYSIS) sortiert. Als Leerwert werden hier 2 Stunden vor Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten Average) und als Textdatei auf einen Datenträger übertragen. Die so vorsortierten und komprimierten Messwerte werden in Excel-Vorlagen übertragen und tabellarisch dargestellt. Die Ablage der erhobenen Daten erfolgt pro Versuchstag in einem eigenen Ordner, der die Versuchsnummer trägt. Ergebnisse und Versuchsprotokolle werden in Papierform nach Nummern sortiert in Ordnern abgelegt.

### Literatur

Klaus Witte, Kai Hu, Johanna Swiatek, Claudia Müssig, Georg Ertl and Björn Lemmer: Experimental heart failure in rats: effects on cardiovascular circadian rhythms and on myocardial β-adrenergic signaling. Cardiovasc Res 47 (2): 203-405, 2000; Kozo Okamoto: Spontaneous hypertension in rats. Int Rev Exp Pathol 7: 227- 270, 1969; Maarten van den Buuse: Circadian Rhythms of Blood Pressure, Heart Rate, and Locomotor Activity in Spontaneously Hypertensive Rats as Measured With Radio-Telemetry. Physiology & Behavior 55(4): 783-787, 1994

### B-4. Bestimmung pharmakokinetischer Kenngrößen nach intravenöser und oraler Gabe

Die pharmakokinetischen Parameter der Substanz werden in männlichen CD-1-Mäusen, männlichen Wister-Ratte und weiblichen Beagle-Hunden bestimmt. Das Applikationsvolumen beträgt bei Mäusen 1 mL/kg, bei Ratten 5 mL/kg und bei Hunden 0.5 mL/kg. Die intravenöse Gabe erfolgt bei Mäusen und Ratten mittels einer speziesspezifischen Plasma/DMSO-Formulierung (99/1) und bei Hunden mittels Wasser/PEG400/Ethanol (50/40/10). Den Ratten wird zur vereinfachten Blutabnahme vor der Substanzgabe ein Silikonkatheter in die rechte *Vena jugularis externa* gelegt. Die Operation erfolgt einen Tag vor dem Versuch unter Isofluran-Narkose und unter Gabe eines Analgetikums (Carprofen, 5 mg/kg s.c.). Die Substanzgabe wird bei Mäusen i.v. bolus und bei Ratten und Hunden mittels 15-minütiger Infusion durchgeführt. Die Blutabnahme erfolgt bei Mäusen nach 0, 0.033, 0.083, 0.17, 0.5, 1, 2, 3, 4, 6, 7 und 24 Stunden sowie bei Hunden und Ratten nach 0, 0.083, 0.25, 0.28 0.33, 0.42, 0.75, 1, 2, 3, 4, 6, 7 und 24 Stunden. Die orale Gabe der gelösten Substanz mittels Schlundsonde wird in allen Spezies basierend auf einer Wasser/PEG400/Ethanol-Formulierung (50/40/10) durchgeführt. Die Blutabnahme in Ratten und Hunden erfolgt hier nach 0, 0.083, 0.17, 0.5, 0.75, 1, 2, 3, 4, 6, 7 und 24 Stunden. Abweichend davon findet die orale Gabe als Suspension mittels einer 0.5%igen Tylose-Formulierung statt. Das Blut wird bei der Entnahme in heparinisierte Röhrchen geleitet. So dann wird mittels Zentrifugation das Blutplasma gewonnen und gegebenenfalls bis zur weiteren Bearbeitung bei -20°C gelagert.

Den unbekannten Proben, Kalibrierproben und QC's wird ein interner Standard zugesetzt (ZK 228859) und es folgt eine Proteinfällung mittels Acetonitril im Überschuss. Nach Zugabe eines Natriumacetat-Puffers (0.01 M, pH 6.8) und folgendem Vortexen wird bei 1000 g zentrifugiert und der Überstand mittels LC-MS/MS (API 4000, AB Sciex) vermessen. Die chromatographische Trennung wird auf einer 1100-HPLC von Agilent durchgeführt. Das Injektionsvolumen beträgt 10 µL. Als Trennsäule wird eine auf 40°C temperierte Luna 5µ C8(2) 100A 50x2mm der Fa. Phenomenex verwendet. Es wird ein binärer Laufmittelgradient bei 400 µL/Min gefahren (A: 0.01M Ammoniumacetat-Puffer pH 6.8, B: 0.1 % Ameisensäure in Acetonitril): 0 Min. (90 % A), 1 Min. (90 % A), 3.50 Min. (15 % A), 4.50 Min (15 % A), 4.60 (90 % A), 7 Min (90 % A). Die Temperatur der Turbo V Ionenquelle beträgt 500°C. Folgende MS-Geräteparameter werden benutzt: Curtain Gas 20 units, Ionsprayvoltage 5 kV, Gas 1/2 35 units, CAD Gas 40 units. Die Quantifizierung der Substanzen erfolgt anhand der Peakhöhen oder -flächen aus extrahierten Ionenchromatogrammen spezifischer MRM-Experimente.

Aus den ermittelten Plasmakonzentration-Zeit-Verläufen werden die pharmakokinetischen Kenngrößen wie AUC, Cₘₐₓ, t_{1/2} (Halbwertszeit) und CL (Clearance) mittels des validierten pharmakokinetischen Rechenprogramms KinEx (Vers. 2.5 und 3) berechnet.

Da die Substanzquantifizierung in Plasma durchgeführt wird, muss die Blut/Plasma-Verteilung der Substanz bestimmt werden, um die pharmakokinetischen Parameter entsprechend anpassen zu können. Dazu wird eine definierte Menge Substanz in heparinisiertem Vollblut der entsprechenden Spezies für 20 Min. im Taumelrollenmischer inkubiert. Nach Zentrifugation bei 1000g wird die Konzentration im Plasma gemessen (s.o.) und durch Quotientenbildung der c_{b}/cₚ-wert ermittelt.

Nach intravenöser Gabe von 0.3 mg/kg von Beispiel 1 in Ratten wurden folgende Werte erhoben:

| Beispiel | 1 |
|---|---|
| AUC ₙₒᵣₘ [kg*h/L] | 4.62 |
| C_{L Blut} [L/h/kg] | 0.33 |
| t_{1/2} [h] | 1.38 |

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 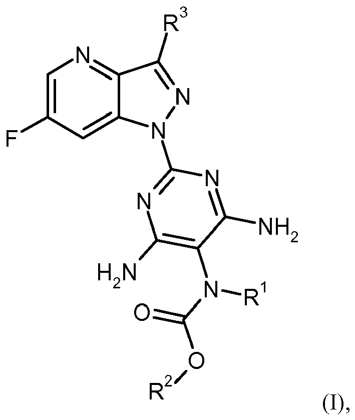 in welcher
R¹ für Wasserstoff oder (C₁-C₄)-Alkyl steht,
wobei (C₁-C₄)-Alkyl mit ein oder zwei Substituenten unabhängig voneinander ausgewählt aus der Gruppe Fluor und Trifluormethyl substituiert sein kann,
R² für (C₁-C₄)-Alkyl, (C₃-C₇)-Cycloalkyl oder einen 4- bis 7-gliedrigen Heterocyclus steht,
wobei (C₁-C₄)-Alkyl mit ein oder zwei Substituenten unabhängig voneinander ausgewählt aus der Gruppe Fluor, Trifluormethyl und (C₃-C₇)-Cycloalkyl substituiert sein kann,
R³ für (C₁-C₆)-Alkyl oder Benzyl steht,
wobei (C₁-C₆)-Alkyl mit einem Substituenten Trifluormethyl substituiert ist,
wobei (C₁-C₆)-Alkyl mit 1 bis 3 Substituenten Fluor substituiert sein kann,
und
wobei Benzyl mit 1 bis 3 Substituenten Fluor substituiert ist,
sowie ihre *N*-Oxide, Salze, Solvate, Salze der *N*-Oxide und Solvate der *N*-Oxide und Salze.

2. Verbindung der Formel (I) nach Anspruch 1, in welcher
R¹ für Wasserstoff, Methyl oder Ethyl steht,
wobei Methyl mit einem Substituenten Trifluormethyl substituiert sein kann,
R² für Methyl, Ethyl, iso-Propyl, Cyclopropyl, Cyclobutyl oder Oxetanyl steht,
wobei Methyl und Ethyl mit ein oder zwei Substituenten unabhängig voneinander ausgewählt aus der Gruppe Fluor, Trifluormethyl und Cyclopropyl substituiert sein können,
R³ für 3,3,3-Trifluorprop-1-yl, 3,3,4,4,4-Pentafluorbut-1-yl oder Benzyl steht,
wobei Benzyl mit 1 oder 2 Substituenten Fluor substituiert ist,
sowie ihre Salze, Solvate und Solvate der Salze.

3. Verbindung der Formel (I) nach Anspruch 1 oder 2, in welcher
R¹ für Wasserstoff, Methyl, Ethyl oder 2,2,2-Trifluorethyl steht,
R² für Methyl, Ethyl oder Cyclopropylmethyl steht,
R³ für 2-Fluorbenzyl steht, sowie ihre Salze, Solvate und Solvate der Salze.

4. Verfahren zur Herstellung von Verbindungen der Formel (I), wie in den Ansprüchen 1 bis 3 definiert, **dadurch gekennzeichnet, dass** man eine Verbindung der Formel (II) 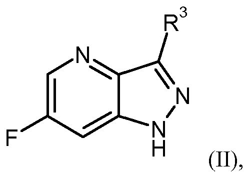 in welcher R³ die in den Ansprüchen 1 bis 3 angegebene Bedeutung hat,
[A] in einem inerten Lösungsmittel in Gegenwart einer geeigneten Base mit der Verbindung der Formel (III) 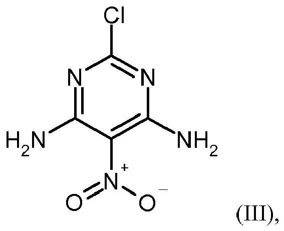 zu einer Verbindung der Formel (IV) 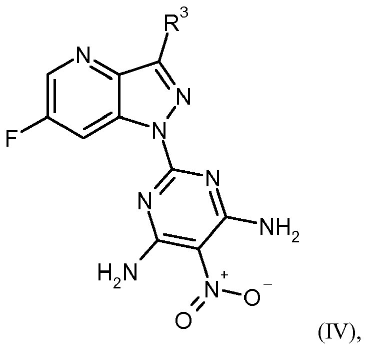 in welcher R³ die in den Ansprüchen 1 bis 3 angegebene Bedeutung hat,
umsetzt, diese dann in einem inerten Lösungsmittel mit einem geeigneten Reduktionsmittel zu einer Verbindung der Formel (V) 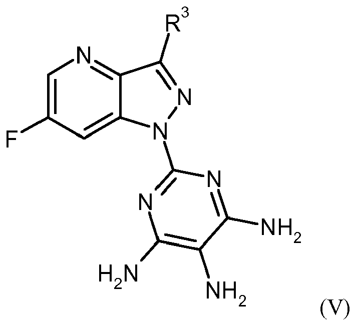 in welcher R³ die in den Ansprüchen 1 bis 3 angegebene Bedeutung hat,
reduziert, diese im Anschluss in Gegenwart einer geeigneten Base mit oder ohne Lösungsmittel mit einer Verbindung der Formel (VI) 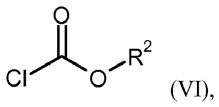 in welcher R² die in den Ansprüchen 1 bis 3 angegebene Bedeutung hat,
zu einer Verbindung der Formel (I-A) 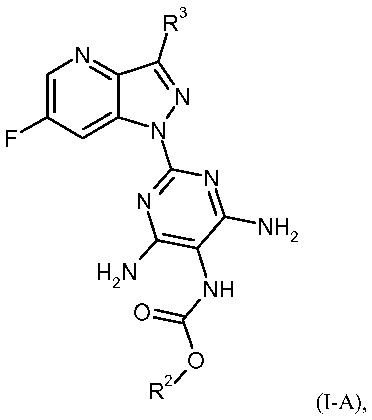 in welcher R² und R³ jeweils die in den Ansprüchen 1 bis 3 angegebenen Bedeutungen haben,
umsetzt,
oder
[B] eine Verbindung der Formel (II) in einem inerten Lösungsmittel unter sauren Bedingungen mit Aminoacetonitril in eine Verbindung der Formel (VII) 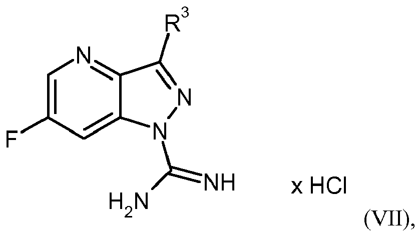 in welcher R³ die in den Ansprüchen 1 bis 3 angegebene Bedeutung hat,
überführt, diese im Folgenden in einem inerten Lösungsmittel in Gegenwart einer geeigneten Base mit der Verbindung der Formel (VIII) 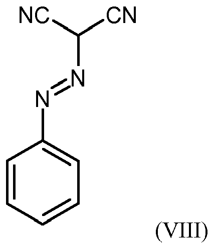 zu einer Verbindung der Formel (IX) 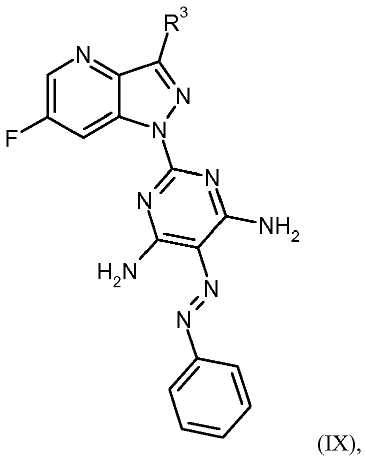 in welcher R³ die in den Ansprüchen 1 bis 3 angegebene Bedeutung hat,
reagiert, und diese anschliessend in einem inertem Lösungsmittel in Gegenwart eines geeigneten Reduktionsmittels zur einer Verbindung (V) reduziert, und diese im Folgenden nach Verfahren [A] weiter zu einer Verbindung (I-A) umsetzt,
oder
[C] eine Verbindung der Formel (I-A) in einem inerten Lösungsmittel mit einer Verbindung der Formel (X)
R¹-X¹ (X),
in welcher R¹ die in den Ansprüchen 1 bis 3 angebene Bedeutung hat und
X¹ für eine geeignete Abgangsgruppe, wie beispielsweise Halogen, Tosylat oder Mesylat, insbesondere Brom oder Iod, steht,
zu einer Verbindung der Formel (I-B) 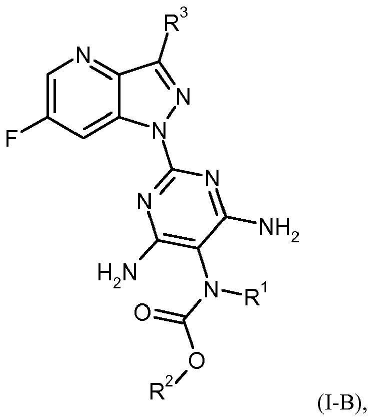 in welcher R¹, R² und R³ jeweils die in den Ansprüchen 1 bis 3 angegebenen Bedeutungen haben,
umsetzt,
und die resultierenden Verbindungen der Formeln (I-A) und (I-B) gegebenenfalls mit den entsprechenden (i) Lösungsmitteln und/oder (ii) Säuren oder Basen in ihre Solvate, Salze und/oder Solvate der Salze überführt.

5. Verbindung der Formel (I), wie in einem der Ansprüche 1 bis 3 definiert, zur Behandlung und/oder Prophylaxe von Krankheiten.

6. Verbindung der Formel (I), wie in einem der Ansprüche 1 bis 3 definiert, zur Verwendung in einem Verfahren zur Behandlung und/ oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

7. Verwendung einer Verbindung der Formel (I), wie in einem der Ansprüche 1 bis 3 definiert, zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

8. Arzneimittel enthaltend eine Verbindung der Formel (I), wie in einem der Ansprüche 1 bis 3 definiert, in Kombination mit einem inerten, nicht-toxischen, pharmazeutisch geeigneten Hilfsstoff.

9. Arzneimittel enthaltend eine Verbindung der Formel (I), wie in einem der Ansprüche 1 bis 3 definiert, in Kombination mit einem weiteren Wirkstoff ausgewählt aus der Gruppe bestehend aus organischen Nitraten, NO-Donatoren, cGMP-PDE-Inhibitoren, antithrombotisch wirkenden Mitteln, den Blutdruck senkenden Mitteln sowie den Fettstoffwechsel verändernden Mitteln.

10. Arzneimittel nach Anspruch 8 oder 9 zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

## Claims

1. Compound of the formula (I) 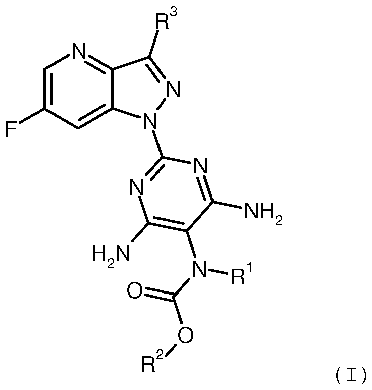 in which
R¹ represents hydrogen or (C₁-C₄)-alkyl,
where (C₁-C₄)-alkyl may be substituted by one or two substituents independently of one another selected from the group consisting of fluorine and trifluoromethyl,
R² represents (C₁-C₄)-alkyl, (C₃-C₇)-cycloalkyl or a 4- to 7-membered heterocycle,
where (C₁-C₄)-alkyl may be substituted by one or two substituents independently selected from the group consisting of fluorine, trifluoromethyl and (C₃-C₇)-cycloalkyl,
R³ is (C₁-C₆)-alkyl or benzyl,
where (C₁-C₆)-alkyl is substituted by one trifluoromethyl substituent,
where (C₁-C₆)-alkyl may be substituted by 1 to 3 fluorine substituents,
and
where benzyl is substituted by 1 to 3 fluorine substituents,
and the *N*-oxides, salts, solvates, salts of *N-*oxides and solvates of the *N*-oxides and salts thereof.

2. Compound of the formula (I) according to Claim 1 in which
R¹ represents hydrogen, methyl or ethyl,
where methyl may be substituted by a trifluoromethyl substituent,
R² represents methyl, ethyl, isopropyl, cyclopropyl, cyclobutyl or oxetanyl,
where methyl and ethyl may be substituted by one or two substituents independently of one another selected from the group consisting of fluorine, trifluoromethyl and cyclopropyl,
R³ represents 3,3,3-trifluoroprop-1-yl, 3,3,4,4,4-pentafluorobut-1-yl or benzyl,
where benzyl is substituted by 1 or 2 fluorine substituents,
and the salts, solvates and solvates of the salts thereof.

3. Compound of the formula (I) according to Claim 1 or 2 in which
R¹ represents hydrogen, methyl, ethyl or 2,2,2-trifluoroethyl,
R² represents methyl, ethyl or cyclopropylmethyl,
R³ represents 2-fluorobenzyl,
and the salts, solvates and solvates of the salts thereof.

4. Process for preparing compounds of the formula (I) as defined in Claims 1 to 3, **characterized in that** a compound of the formula (II) 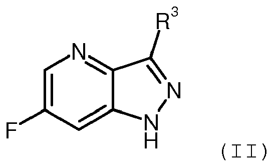 in which R³ has the meaning given in any of Claims 1 to 3
[A] is reacted in an inert solvent in the presence of a suitable base with the compound of the formula (III) 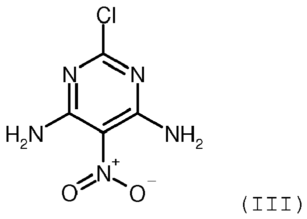 to give a compound of the formula (IV) 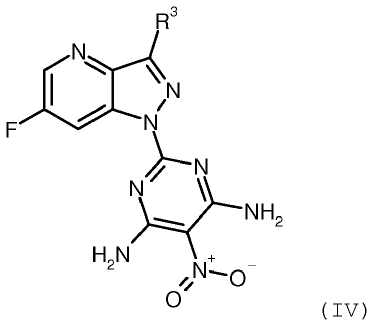 in which R³ has the meaning given in any of Claims 1 to 3,
this is then reduced in an inert solvent with a suitable reducing agent to give a compound of the formula (V) 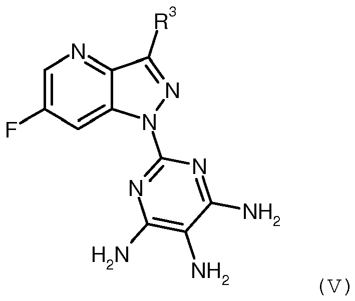 in which R³ has the meaning given in any of Claims 1 to 3,
and this is then reacted in the presence of a suitable base in the presence or absence of a solvent with a compound of the formula (VI) 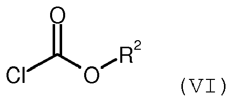 in which R² has the meaning given in any of Claims 1 to 3
to give a compound of the formula (I-A) 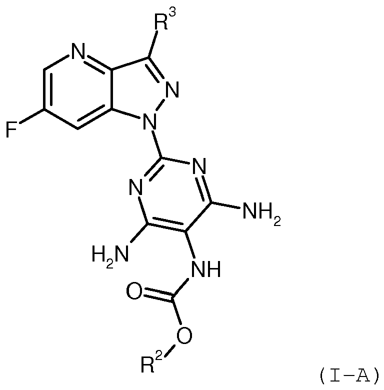 in which R² and R³ are each as defined in any of Claims 1 to 3,
or
[B] a compound of the formula (II) is converted in an inert solvent under acidic conditions with aminoacetonitrile into a compound of the formula (VII) 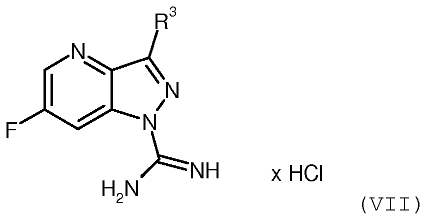 in which R³ has the meaning given in any of Claims 1 to 3,
this is subsequently reacted in an inert solvent in the presence of a suitable base with the compound of the formula (VIII) 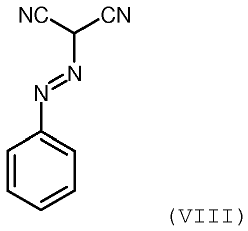 to give a compound of the formula (IX) 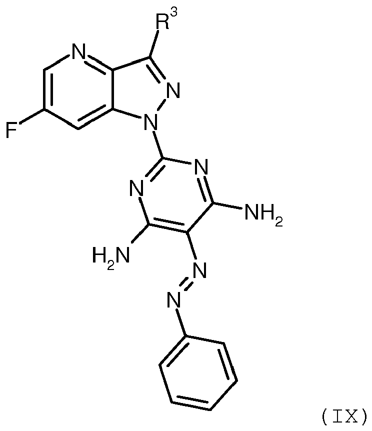 in which R³ has the meaning given in any of Claims 1 to 3,
and this is then reduced in an inert solvent in the presence of a suitable reducing agent to give a compound (V), and this is subsequently reacted further according to process [A] to give a compound (I-A),
or
[C] a compound of the formula (I-A) is reacted in an inert solvent with a compound of the formula (X)
R¹-X¹ (X)
in which R¹ is as defined in any of Claims 1 to 3 and
X¹ is a suitable leaving group, for example halogen, tosylate or mesylate, especially bromine or iodine,
to give a compound of the formula (I-B) 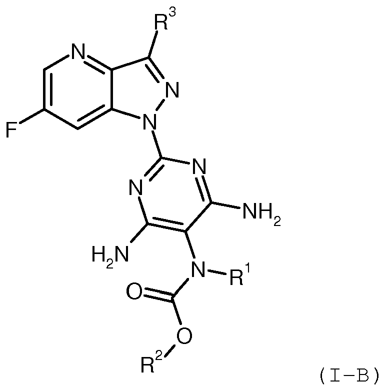 in which R¹, R² and R³ are each as defined in any of Claims 1 to 3,
and the resulting compounds of the formulae (I-A) and (I-B) are, where appropriate, converted with the appropriate (i) solvents and/or (ii) acids or bases into their solvates, salts and/or solvates of the salts.

5. Compound of the formula (I) as defined in any of Claims 1 to 3 for the treatment and/or prophylaxis of diseases.

6. Compound of the formula (I) as defined in any of Claims 1 to 3 for use in a method for the treatment and/or prophylaxis of heart failure, angina pectoris, hypertension, pulmonary hypertension, ischemias, vascular disorders, renal insufficiency, thromboembolic disorders, fibrotic disorders and arteriosclerosis.

7. Use of a compound of the formula (I) as defined in any of Claims 1 to 3 for the preparation of a medicament for the treatment and/or prophylaxis of heart failure, angina pectoris, hypertension, pulmonary hypertension, ischemias, vascular disorders, renal insufficiency, thromboembolic disorders, fibrotic disorders and arteriosclerosis.

8. Medicament comprising a compound of the formula (I) as defined in any of Claims 1 to 3 in combination with an inert, non-toxic, pharmaceutically suitable auxiliary.

9. Medicament comprising a compound of the formula (I) as defined in any of Claims 1 to 3 in combination with a further active compound selected from the group consisting of organic nitrates, NO donors, cGMP-PDE inhibitors, antithrombotic agents, hypotensive agents and lipid metabolism modifiers.

10. Medicament according to Claim 8 or 9 for the treatment and/or prophylaxis of heart failure, angina pectoris, hypertension, pulmonary hypertension, ischemias, vascular disorders, renal insufficiency, thromboembolic disorders, fibrotic disorders and arteriosclerosis.

## Revendications

1. Composé de formule (I) 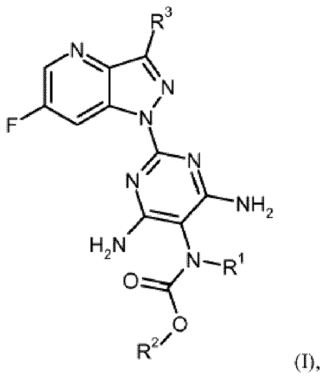 dans laquelle
R¹ représente un atome d'hydrogène ou un groupe alkyle en C₁-C₄,
le groupe alkyle en C₁-C₄ pouvant être substitué par un ou deux substituants choisis indépendamment l'un de l'autre dans le groupe constitué par fluoro et trifluorométhyle,
R² représente un groupe alkyle en C₁-C₄, cycloalkyle en C₃-C₇ ou un hétérocycle à 4 à 7 chaînons,
le groupe alkyle en C₁-C₄ pouvant être substitué par un ou deux substituants choisis indépendamment l'un de l'autre dans le groupe constitué par fluoro, trifluorométhyle et cycloalkyle en C₃-C₇,
R³ représente un groupe alkyle en C₁-C₆ ou benzyle,
le groupe alkyle en C₁-C₆ étant substitué par un substituant trifluorométhyle,
le groupe alkyle en C₁-C₆ pouvant être substitué par 1 à 3 substituants fluoro,
et
le groupe benzyle étant substitué par 1 à 3 substituants fluoro,
ainsi que ses N-oxydes, sels, produits de solvatation, sels des N-oxydes et produits de solvatation des N-oxydes et sels.

2. Composé de formule (I) selon la revendication 1, dans lequel
R¹ représente un atome d'hydrogène, le groupe méthyle ou éthyle,
le groupe méthyle pouvant être substitué par un substituant trifluorométhyle,
R² représente le groupe méthyle, éthyle, isopropyle, cyclopropyle, cyclobutyle ou oxétanyle,
les groupes méthyle et éthyle pouvant être substitués par un ou deux substituants choisis indépendamment l'un de l'autre dans le groupe constitué par fluoro, trifluorométhyle et cyclopropyle,
R³ représente le groupe 3,3,3-trifluoroprop-1-yle, 3,3,4,4,4-pentafluorobut-1-yle ou benzyle,
le groupe benzyle étant substitué par 1 ou 2 substituants fluoro,
ainsi que ses sels, produits de solvatation et produits de solvatation des sels.

3. Composé de formule (I) selon la revendication 1 ou 2, dans lequel
R¹ représente un atome d'hydrogène, le groupe méthyle, éthyle ou 2,2,2-trifluoroéthyle,
R² représente le groupe méthyle, éthyle ou cyclopropylméthyle,
R³ représente le groupe 2-fluorobenzyle,
ainsi que ses sels, produits de solvatation et produits de solvatation des sels.

4. Procédé pour la préparation des composés de formule (I), tels que définis dans les revendications 1 à 3, **caractérisé en ce qu'**on fait réagir un composé de formule (II) 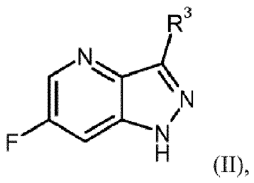 dans laquelle R³ a la signification indiquée dans les revendications 1 à 3,
[A] dans un solvant inerte, en présence d'une base convenable, avec le composé de formule (III) 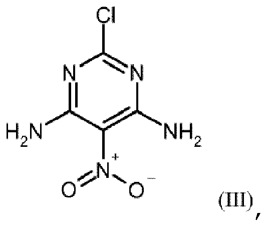 pour obtenir un composé de formule (IV) 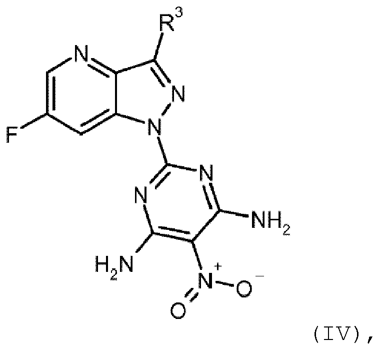 dans laquelle R³ a la signification indiquée dans les revendications 1 à 3,
puis on réduit ce dernier, dans un solvant inerte, à l'aide d'un réducteur convenable, pour aboutir à un composé de formule (V) 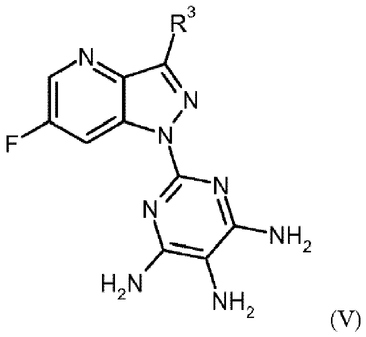 dans laquelle R³ la signification indiquée dans les revendications 1 à 3,
ensuite on fait réagir ce dernier, en présence d'une base convenable, avec ou sans solvant, avec un composé de formule (VI) 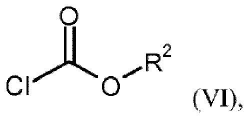 dans laquelle R² a la signification indiquée dans les revendications 1 à 3,
pour obtenir un composé de formule (I-A), 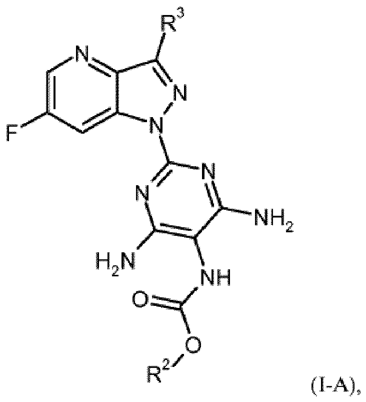 dans laquelle R² et R³ ont chacun les significations indiquées dans les revendications 1 à 3,
ou
[B] on convertit un composé de formule (II) dans un solvant inerte, dans des conditions acides, à l'aide d'aminoacétonitrile, en un composé de formule (VII) 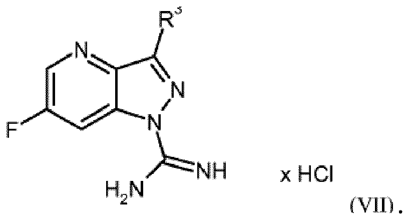 dans laquelle R³ la signification indiquée dans les revendications 1 à 3,
ensuite on fait réagir ce dernier dans un solvant inerte, en présence d'une base convenable, avec le composé de formule (VIII) 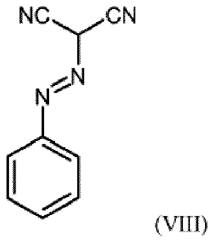 pour obtenir un composé de formule (IX) 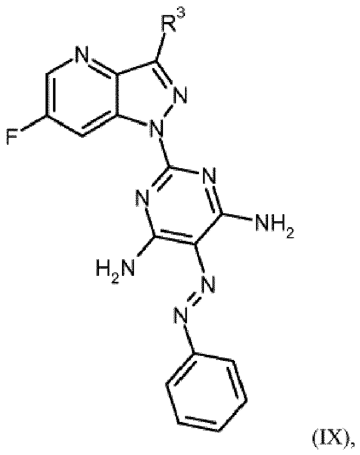 dans laquelle R³ la signification indiquée dans les revendications 1 à 3,
et ensuite on réduit ce dernier dans un solvant inerte, en présence d'un réducteur convenable, pour aboutir à un composé (V), et on poursuit la réaction de ce dernier selon le procédé (A), pour aboutir à un composé (I-A),
ou
[C] on fait réagir un composé de formule (I-A), dans un solvant inerte, avec un composé de formule (X)
R¹-X¹ (X),
dans laquelle R¹ la signification indiquée dans les revendications 1 à 3 et
X¹ représente un groupe partant, comme par exemple halogéno, tosylate ou mésylate, en particulier bromo ou iodo,
pour aboutir à un composé de formule (I-B) 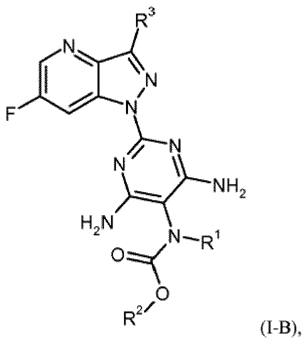 dans laquelle R¹, R² et R³ ont respectivement les significations indiquées dans les revendications 1 à 3,
et éventuellement on convertit les composés de formules (I-A) et (I-B) résultants, à l'aide (i) des solvants correspondants et/ou (ii) des acides correspondants ou des bases correspondantes, en leurs produits de solvatation, sels et/ou produits de solvatation des sels.

5. Composé de formule (I), tel que défini dans l'une quelconque des revendications 1 à 3, destiné au traitement et/ou la prophylaxie de maladies.

6. Composé de formule (I), tel que défini dans l'une quelconque des revendications 1 à 3, pour utilisation dans un procédé destiné au traitement et/ou à la prophylaxie de l'insuffisance cardiaque, de l'angine de poitrine, de l'hypertonie, de l'hypertonie pulmonaire, d'ischémies, de maladies vasculaires, de l'insuffisance rénale, de maladies thromboemboliques, de maladies fibreuses et de l'artériosclérose.

7. Utilisation d'un composé de formule (I), tel que défini dans l'une quelconque des revendications 1 à 3, pour la fabrication d'un médicament destiné au traitement et/ou à la prophylaxie de l'insuffisance cardiaque, de l'angine de poitrine, de l'hypertonie, de l'hypertonie pulmonaire, d'ischémies, de maladies vasculaires, de l'insuffisance rénale, de maladies thromboemboliques, de maladies fibreuses et de l'artériosclérose.

8. Médicament contenant un composé de formule (I), tel que défini dans l'une quelconque des revendications 1 à 3, en association avec un adjuvant inerte, non toxique, pharmaceutiquement convenable.

9. Médicament contenant un composé de formule (I), tel que défini dans l'une quelconque des revendications 1 à 3, en association avec une autre substance active choisie dans le groupe constitué par des nitrates organiques, des donneurs de NO, des inhibiteurs de GMPc-PDE, des agents à activité antithrombotique, des hypotenseurs ainsi que des agents modifiant le métabolisme.

10. Médicament selon la revendication 8 ou 9, destiné au traitement et/ou à la prophylaxie de l'insuffisance cardiaque, de l'angine de poitrine, de l'hypertonie, de l'hypertonie pulmonaire, d'ischémies, de maladies vasculaires, de l'insuffisance rénale, de maladies thromboemboliques, de maladies fibreuses et de l'artériosclérose.
